(19) Europäisches Patentamt
European Patent Office
Office européen des brevets

(11) **EP 4 692 318 A1**

(12) **EUROPEAN PATENT APPLICATION**
published in accordance with Art. 153(4) EPC

(43) Date of publication:
**11.02.2026 Bulletin 2026/07**

(21) Application number: **23933629.0**

(22) Date of filing: **30.06.2023**

(51) International Patent Classification (IPC):
**C12N 5/071** (2010.01)   **A61L 27/50** (2006.01)
**A61L 27/56** (2006.01)   **A61L 27/40** (2006.01)
**A61L 27/38** (2006.01)

(86) International application number:
**PCT/CN2023/104699**

(87) International publication number:
**WO 2024/216756 (24.10.2024 Gazette 2024/43)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.04.2023 CN 202310435265**

(71) Applicants:
• **Roumai Medical (Shenzhen) Co., Ltd
Shenzhen, Guangdong 518000 (CN)**

• **Roumai Medical (Switzerland) SA
Lausanne 1000-1018 (CH)**

(72) Inventors:
• **CHENG, Shiyu
Shenzhen, Guangdong 518000 (CN)**
• **QIAN, Junyan
Shenzhen, Guangdong 518000 (CN)**
• **GAO, Jiajie
Shenzhen, Guangdong 518000 (CN)**

(74) Representative: **Pelanda, Paolo
De Gaspari Osgnach s.r.l.
Via Altinate, 33
35121 Padova (IT)**

(54) **THREE-DIMENSIONAL ARTIFICIAL TUBULAR TISSUE, PREPARATION METHOD THEREFOR, AND USE THEREOF**

(57)    Provided are a three-dimensional artificial tubular tissue, a preparation method therefor, and a use thereof, relating to the technical field of tissue engineering. The method comprises the following steps: (1) preparing a rollable scaffold; (2) seeding cells on the scaffold; and (3) rolling the scaffold into a three-dimensional tubular structure and carrying out *in-vitro* culturing. A three-dimensional topological structure of the scaffold is designed according to different tubular tissue structures, to realize the three-dimensional growth of cells, so that seeded cells can achieve an excellent three-dimensional growth effect directly in the three-dimensional topological structure, and in a scaffold rolling process, each turn of the rolled scaffold can provide an enough tissue thickness for the current cell tissue, so that no cavity would be present between tissues of the same type, and the cells can be better adhered and grown. The method well solves the problems in existing three-dimensional artificial tubular tissues that cavities between cell layers cannot be removed and bleeding and arterial dissections are prone to occur, and avoids the risk of causing aneurysms.

Figure 1

## Description

### TECHNICAL FIELD

The present disclosure belongs to the technical field of tissue engineering, and in particular, relates to a three-dimensional artificial tubular tissue, a preparation method therefor, and use thereof.

### BACKGROUND

Tissue engineering is an interdisciplinary subject that studies the structure, function, and growth mechanisms of biological tissues through materials engineering and life sciences, in order to develop biological substitutes that can repair, maintain, or improve the function of damaged tissues. Tissue engineering has great development prospects in the field of biomedicine. The main goal of tissue engineering is to achieve the repair and regeneration of damaged tissues. Accordingly, a pivotal challenge in this subject lies in cultivating cell scaffolds with highly biomimetic structures *in vitro* and ensuring their successfully integration and reconstruction into artificial tissues and organs with excellent structure and function upon transplantation *in vivo.*

There are extensively distributed biological systems with tubular structures in the human body, mainly including the vascular system, digestive system, respiratory system, nervous system and reproductive system. Common tubular tissues include the trachea, blood vessels, lymphatic vessels, esophagus, nerve conduits, urethra, intestines and reproductive tracts. These hollow organs and tissues drive various bodily functions and perform their own different functions. Therefore, three-dimensional artificial tubular tissues also exhibit various structures and functions. When constructing various biomimetic tubular tissues using tissue engineering techniques, it is necessary to consider the structural and functional differences among various tubular tissues.

To better fabricate various biomimetic artificial tubular grafts that possess structural and functional similarities to natural ones, it is first necessary to gain a comprehensive understanding of the structures of various human tubular tissues and organs. The following content primarily provides separate introductions focusing on the vascular system, digestive system, respiratory system and nervous system.

The primary function of the vascular system is to transport blood throughout the body at a relatively high velocity, generating significant fluid shear stress on the blood vessel walls. Blood vessels are mainly composed of three layers of membranes: endothelium, media and adventitia, with a hollow lumen at their center. The vascular endothelium is relatively permeable, allowing biochemical factors to be transported across the vessel wall and even across cells in certain disease states. The vascular media is composed of circumferentially dense vascular smooth muscle cells (VSMCs) and elastin, and its main function is to withstand the expansion and impact forces caused by blood flow. The vascular adventitia is mainly composed of collagen and fibroblasts. The vascular adventitia is a loose connective tissue whose function is to wrap and support the blood vessels, while giving the blood vessels a certain tensile strength. Collagen in blood vessels gives them a certain strength, and elastin makes blood vessels elastic. Blood vessels vary greatly in size ranging from capillaries and microvessels with a diameter of only a few micrometers to veins and arteries with a diameter of up to 30 mm. Small capillaries, such as those forming the blood-brain barrier, are composed of a single cell, wrapping around itself to form an internal lumen. Therefore, the differences in tubular diameter and structural among various blood vessels make the construction of tissue-engineered blood vessels more complicated. Currently, mainly large-caliber artificial vascular tissues have been fabricated, while small-caliber artificial vascular tissues requires further research to overcome existing challenges.

The digestive system is mainly composed of the gastrointestinal tract. Each segment of the gastrointestinal tract is composed of four layers, starting from the luminal layer, including the mucosa, submucosa, muscularis propria and serosa. The mucosa contains intestinal epithelial cells and performs absorptive, secretory and protective functions. The intestinal epithelium serves as a tight barrier system that separates the contents inside the lumen from the surrounding tissues. The intestinal epithelium has a complex 3D structure with lumen protrusions (called villi) and central invaginations (called crypts). This 3D structure maximizes the internal surface area and facilitates nutrient absorption. The submucosa houses a lineage of immune cells, nerves, and lymphocytes, and is surrounded by the muscularis propria, which provides a peristaltic pump through muscle cells, performing the key function of intestinal motility. The outermost layer is the serosa (or adventitia), which forms a protective barrier around the gastrointestinal tract. Additionally, the intestinal tract is equipped with a sophisticated network of nerves called the enteric nervous system, which includes two parallel plexuses, the submucosal plexus and the myenteric plexus. Therefore, the sophisticated structure of the gastrointestinal tract further complicates the design of artificial digestive tract tissue.

The respiratory system includes the upper respiratory tract and the lower respiratory tract, with the trachea serving as the foundation for swallowing, speaking and breathing and being located below the upper respiratory tract which includes the nasal cavity, throat, and pharynx and is part of the lower respiratory tract which includes the trachea, bronchi, bronchioles, and alveoli. The trachea is mainly composed of the following four layers: mucosa, submucosa, hyaline cartilage and adventitia. The mucosa contains a pseudostratified epithelium that lines in the lumen and contains a variety of cell types. The submucosa is a layer of connective tissue that contains submucosal glands that aid in the secretion of mucus. The hyaline cartilage layer consists of a horseshoe-shaped ring of hyaline cartilage connected by fibroelastic tissue. The posterior side of the fibroelastic tissue is closed by a membranous structure composed of longitudinally oriented smooth muscle. Finally, the adventitia is composed of connective tissue, and both the hyaline cartilage and adventitia layer are fundamental to the unique structural and mechanical properties of the trachea. The adventitia also contains many other cell types, such as fibroblasts, adipocytes, nerves, and connections to the vascular system, which are necessary to meet blood, nutritional, and metabolic needs. Unlike *in vitro* models of the vascular system and intestinal tract, the trachea is susceptible to a range of airway disorders caused by infection, stenosis, collapse, or cancer. To date, tracheal tissue engineering has mainly focused on implantable scaffolds for tracheal replacement.

The nervous system is vital to the human body, responsible for regulating the body's physiological functions and maintaining the stability of the internal environment. The nervous system is divided into the central nervous system and the peripheral nervous system. The central nervous system mainly provides instructions for the body's comprehensive behavior, and the peripheral nervous system mainly governs the body's sensory and motor functions. Damage to the nervous system will cause loss of sensory abilities such as vision, hearing, and olfaction, or loss of motor abilities such as chewing, walking, and grasping, as well as consciousness disorders such as dementia, dizziness, and amnesia, which will pose a significant threat to human health. The main component of the nervous system is nervous tissue, which is composed of neurons and glial cells. Unlike ordinary cells in organisms, neurons vary greatly in length, ranging from a few microns to one meter. This phenomenon is attributed to two special protrusion structures of neurons, i.e., axons and dendrites. Glia account for a large proportion of neural tissue, their cell bodies also have irregular protrusions, and their primary functions are to support neurons, insulate signals, provide nutrition and protection, etc. The entire nerve exhibits a three-layer structure, and its cross-section appears flat. Therefore, to achieve a high degree of biomimicry in tissue-engineered neural scaffolds, the first step is to simulate the compositional structure of neural tissue.

Although humans have long understood various biological systems and tissues, the process of culturing cells *in vitro* and attempting to construct artificial tissues and organs has still undergone a long developmental journey. Since Willhelm Roux isolated cells from chicken embryos in 1885, the concept of *in vitro* cell culture was established for the first time. Initially, traditional cell culture methods have been cultured on a two-dimensional plane and have gone through a long period of development. However, it was discovered that with this monolayer cell culture method, due to changes in the *in vitro* environment, cells gradually lost their original characteristics during proliferation. This phenomenon is inconsistent with the conditions inside the human body. Cells growing in three-dimensional tissues are significantly different from those in monolayer structures or suspension cultures.

Numerous studies have shown that the environment and behavior of cells in real three-dimensional tissues are far more complex than those on a two-dimensional plane. Compared with two-dimensional planar culture, cells in three-dimensional culture are highly complex in many aspects such as morphology, biochemistry, protein expression, and gene expression. The *in vitro* three-dimensional culture environment is more conducive to the maintenance and development of cell functions than the two-dimensional culture environment. Allowing cells to grow in spatial three-dimensional environment enables them to adopt a growth pattern closer to that *in vivo,* which is more conducive to the formation of structures similar to those of *in vivo* tissues, thereby exerting their functions. Therefore, three-dimensional cell culture technology has emerged as a solution, and it has undergone decades of development to date.

Three-dimensional cell culture (TDCC) technology refers to the *in vitro* co-culture of different material scaffolds with three-dimensional structures with different types of cells, allowing cells to grow and migrate within the three-dimensional structure of the scaffolds, simulating the *in vivo* cell growth environment to the greatest extent and bringing cells closer to the *in vivo* growth pattern. In the first half of the 20th century, scholar Holtfreder and his colleagues pioneered the establishment of an *in vitro* three-dimensional cell culture method, laying the foundation for subsequent three-dimensional culture technology. By the 1950s, scholar Moscona and his colleagues took the lead in using three-dimensional culture methods to study the proliferation, differentiation and aggregation of cancer cells. In recent decades, three-dimensional culture technology has been widely studied and developed, and has been widely used in the field of tumor research.

The construction of artificial tissues generally involves three major factors: seed cells, scaffold materials and *in vitro* construction of cells and materials. However, due to the complex physiological structure and diversity of real human tubular tissues, each performing different functions, it is necessary to integrate multiple factors to create an approximate model of the desired tissue. This poses significant challenges for the development of artificial tubular tissues.

Firstly, the source and type of cells must be considered. Any tubular system requires a source of epithelial cells, yet the exact behavior of these cells depends on the different tissues involved. Epithelial cells are often co-cultured with extracellular matrix (ECM)-producing cells such as fibroblasts or smooth muscle cells, but even the type of matrix-producing cells can vary depending on their use. For example, the trachea requires the use of chondrocytes or mesenchymal stem cells that have differentiated along the chondrogenic pathway. On the other hand, different tissues also require different supporting cells. For example, the native intestinal epithelium contains goblet cells that secrete mucus, and the native blood vessels and intestinal tract have a layer of muscle tissue, which requires the acquisition of appropriate muscle cells.

Secondly, the design of scaffolds is equally challenging. First, a structure needs to be created to support cell growth, and then the desired tissue can be constructed without affecting the overall function of the synthesized tissue. These challenges are particularly prominent in the design of tubular systems. Tubular scaffolds not only need to provide a necessary three-dimensional support structure for cell nutrient acquisition, growth, proliferation, and differentiation metabolism, but also need to provide cells with appropriate chemical, morphological, and topological structural information to guide cells to form target functional tissues. In addition, a favorable physiological microenvironment is also needed to provide for cells to secrete extracellular matrix and ultimately form corresponding tissues or organs. Therefore, the tubular scaffolds need to have an appropriate pore size and porosity to create favorable conditions for the delivery of nutrient metabolites and the ingrowth of seed cells, and have a certain mechanical strength to ensure that the newly formed tissue can maintain an appropriate morphology during regeneration, making it less susceptible to damage from external forces and not causing mechanical damage to the surrounding original tissues. In addition, the scaffold material itself should be widely available and exhibit good biocompatibility, not easily triggering adverse reactions such as coagulation, inflammation and immune rejection, and possessing a certain level of sterilizability, good plasticity and an appropriate degradation rate to adapt to the growth and proliferation rates of different tissues and cells, thereby providing sufficient space for tissue regeneration. This undoubtedly brings many difficulties to the development of tissue-engineered scaffolds.

Thirdly, the integration between cells and scaffold materials is also extremely challenging, mainly due to the following factors: the presence of air in the scaffold, the existence of interfacial forces and surface tension inside the scaffold, the hydrophilic-hydrophobic properties of the scaffold material, and the complex shape of the scaffold. These structural factors lead to problems such as uneven distribution of seeded cells on the scaffold, uncontrollable seeding position, and low seeding efficiency. On the other hand, the complex structure of tubular tissue makes the growth of cells on scaffolds even more difficult. For example, the formation and sustained integrity of epithelial or endothelial barriers ensure the selective permeation of essential nutrients and metabolic by-products while preventing the entry of harmful or pathogenic compounds. Without successful barrier formation and characterization, the functionality and even cell survival of these models will be limited.

With the continuous advancement of tissue engineering research, it has been discovered that although tubular tissues exhibit diverse functions, they share unified structural characteristics. Within the lumen of these tubular tissues, there is a group of barrier-forming cells called epithelial cells (referred to as endothelial cells in the vascular system), with the function being to separate the luminal contents from the surrounding tissues and organs while allowing selective permeability and transport across the epithelial layer. Based on this, when producing tubular scaffolds suitable for various tissue engineering applications, it is first necessary to generate complete tubular openings and form a semipermeable epithelial or endothelial layer.

The primary requirement for tubular scaffolds is the ability to form a contiguous epithelial or endothelial lining, and the most effective approach is to achieve this is uniform seeding of cells on two-dimensional (2D) non-porous surfaces or in injectable media such as hydrogels. However, tubular scaffolds are usually three-dimensional rather than two-dimensional, so uniform seeding of cells on the 3D surface is essential. Some researchers have seeded cells on planar membranes and then rolled these membranes into a tubular shape; other researchers have tried to use dynamic methods, relying on rotation or pressure to drive the uniform attachment of cells to the surrounding walls. Nevertheless, the porous structure of the scaffold makes the formation of a contiguous epithelium more challenging. Other studies have used a layered approach to fill extracellular matrix (ECM)-producing cells or deposited cells back into the wells, aiming to assist in the formation of epithelial cells. Many studies on engraftment drive also rely on *in vivo* cell infiltration, and yet all these methods have certain limitations.

After more than 20 years of development, significant progress has been made in the preparation of tissue-engineered scaffolds. Based on the structural characteristics of tubular tissues, various methods have been developed to fabricate tubular scaffolds, including fiber bonding, solvent casting/particulate leaching, phase separation, in-situ polymerization, emulsion freeze-drying, gas foaming, rapid prototyping, self-assembly and electrospinning.

The above fabrication methods have been widely developed and used in the preparation of various three-dimensional tissues. However, these methods still fail to address numerous issues.

For example, in the case of blood vessels cultured with large-pore, non-woven tubular degradable scaffolds, the tubular scaffolds cannot control the precise spatial distribution of the three types of cells, making it impossible to achieve a more biomimetic three-layered structure and to construct small-caliber blood vessels with a diameter of less than 6 mm and a multi-layered cell structure.

Another example is traditional 3D cell seeding methods: they merely involve immersing a scaffold with a three-dimensional topological structure in a cell suspension of a specific cell concentration, followed by dynamic stimulation culture including rotation and oscillation. Such seeding methods cannot effectively achieve the culture and differentiation of specific cells in a specific topological structure, resulting in the inability to achieve properties similar to real tissues.

For example, cell 3D printers, developed based on 3D printing and microscopy technologies, integrate the overlapping spraying of polymer matrices and cells in solutions, enabling the fabrication of 3D structures. However, to ensure the smooth operation of the printer nozzles, the scaffold materials used in cell 3D printers are mostly hydrogels, which makes it difficult for the printed scaffolds to form tissues with a certain mechanical strength. On the other hand, due to the limitations of hydrogel materials, it is impossible to achieve the biomimetic fabrication of biological structures such as capillaries or villi. In addition, this method cannot achieve high-throughput tissue printing.

In terms of cell seeding, existing methods also have the disadvantages of low cell seeding efficiency and uneven distribution of cells within the scaffold. Although various attempts have been made to improve the efficiency and uniformity of cell seeding, including perfusion seeding and spinner flask reactor seeding, these technologies have limited effects in practical applications and have not been widely used in the field of tissue engineering.

For example, in the biomimetic design of the multi-layered structures of natural blood vessels, significant challenges remain regarding the structural orientation of the scaffold, the formation of the luminal endothelial layer, and the colonization of cells into the interior of the scaffold. Further research is needed to address the following issues: how to improve the long-term patency rate and compliance of tissue-engineered vascular scaffolds; how to develop biomaterials with good bioactivity to reduce the occurrence of thrombosis and infection; how to find the optimal balance between the mechanical strength, degradation rate and tissue formation rate of the scaffold material and how to solve the problems of limited sources of seed cells and long time for constructing autologous blood vessels *in vitro.*

To overcome the above-mentioned defects, researchers have carried out extensive work and developed a series of advanced methods for constructing three-dimensional tubular tissues, and achieved the following results.

Chinese patent document CN 102884172 B provides a system for aseptic isolation, collection of cells, and the seeding of cells onto scaffolds or tissue grafts. The system is capable of isolating and collecting cells from target sources and seeding these cells onto a biocompatible three-dimensional scaffold or tissue graft. The scaffold or graft can be further incubated within the system for culture, transport, storage, and testing, e.g., as a tissue graft, such as a vascular graft, and/or implanted into a subject, to regenerate and/or repair a tissue *in vivo, in vitro* or *ex vivo.* However, the structure of this system is extremely complex and is not suitable for the collection, culture and implantation of all cells, thus still having great limitations in the field of tissue engineering.

Chinese patent document CN 106085847 B provides a scaffold-based cell seeding platform and method based on 3D printing technology, including a three-dimensional motion platform, a control system, a scaffold, an image acquisition system, an image processing system, a host computer and a dual nozzle system. However, the scaffold material on which this cell seeding platform is based is still mainly hydrogel, which has the disadvantages of uncontrollable fine structures and weak mechanical properties of the scaffold.

Chinese patent document CN 108653815 B discloses a three-dimensional rolled structure, which constructs an artificial blood vessel by first performing two-dimensional cell patterning and then conducting self-rolling. However, this structure cannot effectively complete the three-dimensional seeding and construction of cells. When the scaffold has a dense structure, the seeded cells have difficulty penetrating the dense scaffold, resulting in the failure of the single-layer thickness of the finally formed tubular tissue to reach the required thickness. When the scaffold material has a porous structure, cells will form unnecessary excessive migration between layers in the early stage, resulting in the mixing of multiple cells, making it impossible to accurately control the distribution and proliferation of cells in the three-dimensional scaffold material. More importantly, the different cell layers obtained using the claimed method have obvious cavities between the layers, and the same cell tissue has the defect of failing to fuse well after rolling.

Analysis of existing fabrication processes for tubular scaffolds shows that the gaps between endothelial cells are often larger, which increases the chance of vascular smooth muscle cells or growth factors entering the subendothelial space, thereby causing intimal hyperplasia. The fabrication process of these artificial tubular tissues cannot eliminate the cavities between cell layers, resulting in a higher probability of blood leakage and arterial dissection. Blood enters the vessel wall through the intima, causing luminal stenosis and occlusion and leading to the formation of aneurysms.

According to a relevant literature (DOI: https://doi.org/10.7461/jcen.2020.22.3.127, Clinical analysis of young adult patients with ruptured intracranial aneurysms: a single-center study of 113 consecutive patients, J Cerebrovasc Endovasc Neurosurg. 2020; 22(3):127-133. Published online September 21, 2020), a study on intracranial aneurysms in young adult patients (<=40 years old) shows that the mean size of aneurysms is in the range of $6.6\pm3.7$ mm. However, in young adult patients, aneurysms with a size smaller than 5 mm can cause symptoms. The study points out that young adult patients have a higher rupture risk of small aneurysm than middle-aged and elderly patients. In the study, 94.2% of ruptured aneurysms in young adult patients are smaller than 10 mm, and 47.6% are smaller than 5 mm. What is more noteworthy is that the timing of aneurysm rupture in young adult patients is earlier than that in middle-aged and elderly patients. When an aneurysm is initially formed, the thickness of the vessel wall that constitutes the cavity has not yet reached the mechanical strength to support itself. At this stage, if it is frequently exposed to high pressure and high-intensity living environment, it is highly likely to trigger aneurysm rupture. Not only the size of the aneurysm but also its shape has a great impact on the chance of rupture. The literature points out that aneurysms with long or other irregular shapes are more likely to rupture. Meanwhile, smoking is a major risk factor for aneurysms. Currently, the popularity of e-cigarettes has led to a decline in an overall downward shift in the age structure of smoking populations, and aneurysms have accordingly shown an earlier-onset trend.

However, due to the inability of current scaffold fabrication processes to resolve the issue of cavities forming between cell layers, surgical treatment becomes the only option once arterial dissection occurs and causes an aneurysm. The commonly used methods are intracranial aneurysm clipping or endovascular interventional therapy, which seriously limits the application of tissue engineering and causes secondary pain to patients.

Therefore, how to improve the fabrication process of existing tubular tissue-engineered scaffolds and solve the issues of the current processes, such as the presence of cavities between cell layers, the high tendency for blood leakage and arterial dissection, as well as the risk of inducing aneurysms, has become a technical problem that needs to be solved urgently.

## SUMMARY

The objective of the present disclosure is to solve the above technical issues, thereby providing a three-dimensional artificial tubular tissue, a preparation method therefor and use thereof. The technical objective of the present disclosure is to solve the issue that the three-dimensional artificial tubular tissue obtained by existing preparation methods fail to eliminate the cavities or gaps between cell layers, making the prepared tissue-engineered scaffold to be prone to blood leakage and arterial dissection *in vivo,* and easily causing aneurysms.

To achieve the above objectives, the technical solution of the present disclosure is as follows.

One of the objectives of the present disclosure is to provide a method for preparing a three-dimensional artificial tubular tissue.

A method for preparing a three-dimensional artificial tubular tissue, including the following steps:

(1) preparing a rollable scaffold;

(2) seeding cells on the scaffold; and

(3) rolling a product obtained in step (2) into a three-dimensional tubular structure and conducting *in vitro* culture to obtain the three-dimensional artificial tubular tissue;

wherein a method for preparing the rollable scaffold includes any one of the following methods:

Method 1: dividing the rollable scaffold into two different cell seeding regions sequentially from a rolling start end to a rolling termination end; controlling widths of the two different cell seeding regions along a roll direction of the scaffold such that each of the different cell seeding regions forms at least one complete tubular layer after rolling, and the tubular layer located on an outer layer is capable of wrapping the tubular layer located on an inner layer after rolling; and controlling a thickness ratio of the scaffold in the two different cell seeding regions, from thin to thick, to be 1:2-80;

Method 2: dividing the rollable scaffold into three different cell seeding regions sequentially from a rolling start end to a rolling termination end; controlling widths of the three different cell seeding regions along a roll direction of the scaffold such that each of the different cell seeding regions forms at least one complete tubular layer after rolling, and the tubular layer located on an outer layer is capable of sequentially wrapping the tubular layer located on an inner layer after rolling, namely, an outermost layer wraps a middle layer, and the middle layer wraps an innermost layer; and controlling a thickness ratio of the scaffold in the three different cell seeding regions, from thin to thick, to be 1:2-80:2-3000; or

Method 3: dividing the rollable scaffold into four different cell seeding regions sequentially from a rolling start end to a roll end; controlling widths of the four different cell seeding regions along a roll direction of the scaffold such that each of the different cell seeding regions forms at least one complete tubular layer after rolling, and the tubular layer located on an outer layer is capable of sequentially wrapping he tubular layer located on an inner layer after rolling, namely, an outermost layer wraps a second outermost layer, the second outermost layer wraps a middle layer, and the middle layer wraps an innermost layer; and controlling a thickness ratio of the scaffold in the four different cell seeding regions, from thin to thick, to be 1:2-80:2-700:2-3000.

It should be noted that the phrase of "each of the multiple different cell seeding regions forms at least one complete tubular layer after rolling" mentioned in the present disclosure, respectively refers to the following situations: the two different cell seeding regions are rolled to form an outer layer and an inner tubular layer, respectively, with the outer layer enclosing the inner layer; the three different cell seeding regions are rolled to form tubular layers, namely, an outer tubular layer, a middle tubular layer, and an inner tubular layer, respectively, with the outer tubular layer enclosing the middle tubular layer and the middle tubular layer enclosing the inner tubular layer; the four different cell seeding regions are rolled to form tubular layers, namely, an outer tubular layer, a second outer tubular layer, a middle tubular layer and an inner tubular layer, with the outer tubular layer enclosing the second outer tubular layer, the second outer tubular layer enclosing the middle tubular layer, and the middle tubular layer enclosing the inner tubular layer.

In addition, the phrase of "each layer forms at least one complete tubular layer" in the present disclosure means that after rolling, it is not limited to forming only one tubular layer. For example, the inner tubular layer of 1.5, 2, or 2.5 turns may be formed, and the outer layer may wrap the inner layer. In this case, the outer layer may also be a tubular layer of more than one turn, such as a tubular layer of 1.5 or 3 turns. The above situation is also applicable to three-layer and four-layer structures, as long as the tubular layer formed by the cell seeding region in the outer layer after rolling can wrap the adjacent inner layer in sequence.

The method for preparing the above-mentioned three-dimensional artificial tubular tissue provided by the present disclosure is to design a three-dimensional topological structure during the scaffold preparation process. By controlling the thickness ratio of different parts of the scaffold, the two-dimensional cell patterning can be directly replaced with the three-dimensional cell patterning, such that the seeded cells can directly achieve three-dimensional growth in the three-dimensional topological structure. Therefore, during the subsequent rolling process, each turn of the rolled scaffold can provide sufficient tissue thickness for the current cell tissue, such that there will be no cavities between the same tissues, allowing cells to adhere and grow better. The method of the present disclosure solves the issues of existing methods such as the inability to eliminate cavities between cell layers, the high tendency for blood leakage and arterial dissection, and the risk of causing aneurysms by well controlling the thickness ratio of different cell seeding regions during the three-dimensional topological structure design.

The design of the three-dimensional topological structure of the present disclosure can be prepared by different processes such as multi-layer film lamination with needle-punching and carding, or electrospinning and melt-blowing. As long as the corresponding thickness ratio is controlled in accordance with the method of the present disclosure to form a specific three-dimensional topological structure, the three-dimensional growth of cells can be well guaranteed, thereby eliminating the cavity between the cell layers, solving the issue that the artificial tubular tissue is prone to blood leakage and arterial dissection, and will not cause aneurysms.

The three-dimensional tissue structure formed by rolling in the present disclosure is divided into two-layer, three-layer and four-layer structures with different porosity, pore size and fiber diameter. The two-layer structure is suitable for neural tissues, the three-layer structure is suitable for vascular tissues, urethra tissues and vas deferens, and the four-layer structure is suitable for intestinal tissues, esophageal tissues, tracheal tissues and fallopian tubes. The present disclosure can well prepare a variety of three-dimensional tubular tissue structures with various structures and functions.

The contribution of the present disclosure to the existing technologies lies in: providing a three-dimensional artificial tubular tissue without cavities between layers. The artificial tubular tissue does not cause blood leakage or arterial dissection during implantation and will not cause the risk of aneurysm. However, the existing three-dimensional artificial tubular tissues all have cavities between layers, which can easily lead to blood leakage and arterial dissection, and pose a risk of causing aneurysms.

Further, the scaffold of the present disclosure includes a dense scaffold and a loose scaffold, where a porosity and a pore size of the loose scaffold are both larger than those of the dense scaffold. The present disclosure designs a three-dimensional topological structure of the scaffold to consist of a dense scaffold and a loose scaffold. It has been found that the scaffold structure can effectively prevent the migration of cells between different layers and provide sufficient growth space for cells, such that cells can fully grow on the scaffold. By designing the scaffold structure as described above, it is observed that seeded cells could easily invade the loose layer of the scaffold and be evenly distributed in the scaffold gaps. The advantage here is that the cells do not merely remain on the scaffold surface, but can form tissue with the same thickness as the loose scaffold. Secondly, since the seeded cells cannot penetrate the dense layer of the scaffold, the cells in the overlapping part of the scaffold will not migrate between layers before the dense layer degrades during the rolling process. This can fully ensure the thickness of the tissue formed after cell culture, such that the cultured tissue has a controllable thickness and can have multi-layer tissue composed of cells with different functions.

For example, when constructing three-dimensional artificial blood vessels, the result of culturing cells on such a scaffold is that the cell suspension can quickly penetrate into the loose layer, while the cells will not fall through the gaps to the bottom of the seeding vessel during the cell seeding process. The endothelial cells in the suspension can quickly aggregate into cell clusters. The clustered cells can grow adhering to the smooth dense layer without migrating to the smooth muscle layer. The endothelial tissue formed can be smoother, and the decellularized product is more conducive to the differentiation of autologous vascular cells into endothelial cells after implantation.

Further, the loose scaffold of the present disclosure has a thickness of 20-5,000 $\mu$m, a porosity of 90% or more, a pore size of 20-1,000 $\mu$m, and a fiber diameter of 0.05-30 $\mu$m; the dense scaffold has a thickness of 1-200 $\mu$m, a porosity of 59%-90%, a pore size of 0.05-500 $\mu$m, and a fiber diameter of 0.05-30 $\mu$m.

Further, a preparation method for the loose scaffold and the dense scaffold include any one or more of casting, micro-injection molding, blowing molding, extrusion, calendaring, film blowing extrusion, rotational molding, dip coating, 2D printing, hot stamping, melt-blowing, wet spinning, needle spinning, electrospinning, melt spinning, melt-blow spinning, thermal bonding, chemical bonding, spunlacing, needle punching, or other non-woven processes.

Further, materials used for the loose scaffold and the dense scaffold include at least one of poly(glycolic acid) (PGA), poly(lactic acid) (PLA), poly(ε-caprolactone) (PCL), poly(lactide-co-caprolactone) (PLC), poly(p-dioxanone) (PPDO), poly(3-hydroxybutyrate-co-3-hydroxyvalerate) (PHBV), hyaluronic acid (HA), polyurethane (PU), poly(ethylene terephthalate)(PET), polypropylene (PP), polyethylene (PE), polyamide (PA), poly(methyl methacrylate) (PMMA), polytetrafluoroethylene (PTFE), poly(ether sulfone) (PES), poly(ether ether ketone) (PEEK), polystyrene (PS), polypropylene (PP), poly(methyl methacrylate) (PMMA), polycarbonate (PC), poly(glycolide-co-trimethylene carbonate) (PGA-TMC), poly(glycolide-co-ε-caprolactone) (PGA-CL), chitosan or collagen, or copolymer materials thereof.

On the basis of the above, firstly, the present disclosure provides an artificial tubular tissue consisting of a double-layer structure.

Specifically, the three-dimensional artificial tubular tissue with a double-layer structure provided by the present disclosure is a neural tissue, and a preparation method of a scaffold thereof includes: dividing the scaffold into two different cell seeding regions, and controlling a thickness ratio of the scaffold in the two different regions, from thin to thick, to be 1:20-50 from the rolling start end to the rolling termination end.

Further, the scaffold of the neural tissue also includes a double-layered bundled neural structure. The bundled nerves are wrapped by two layers of outer sheath tissue, and the thickness ratio of the bundled neural scaffold is controlled, from thin to thick, to be 1: 12-24 from the rolling start end to the rolling termination end.

Further, a region of the neural scaffold at the rolling start end is also provided with prismatic protrusions distributed along an axial direction, with a distribution density of 10-15 per millimeter and a length of 5-20 mm. Each prismatic protrusion is independent of each other and is separated from each other by a dense layer of 1-8 microns.

Peripheral nerve trauma caused by car accidents, accidents and natural disasters prevents the patient's sensory signals from being transmitted to the brain and spinal cord, and motor commands from the brain cannot be smoothly transmitted to the target organs. Patients who lose the ability to coordinate movements and perceptions in their nervous system will lose the ability to take care of themselves in daily life, causing great trouble to their lives. There are currently two mainstream treatment options: one is to use autologous nerve transplantation, and the other is to use allogeneic acellular nerve transplantation. The former requires a second surgery and requires nerves in other parts of the patient. The latter requires organ or cadaver donations to extract peripheral nerves, making large-scale production impossible and standardization difficult. The technology mentioned in the present disclosure is used to construct an artificial neural tissue scaffold, and by seeding neurons, glial cells and Schwann cells expanded from a cell bank on the scaffold, and using electrical stimulation to guide the nerve axons to extend along the tubular scaffold, the resulting tissue-engineered artificial peripheral nerves can be well used for repairing peripheral nerve trauma after decellularization treatment. There will be no cavities between the layers of the artificial neural tissue constructed by the method of the present disclosure, thus avoiding blood leakage and arterial dissection, and eliminating risk of causing aneurysms.

In addition, the three-dimensional artificial tubular tissue with a double-layer structure provided by the present disclosure also includes a medium-caliber arterial vascular tissue, and a preparation method of a scaffold thereof includes: dividing the scaffold into two different cell seeding regions, and controlling the thickness ratio of the scaffold in the two different regions from the rolling start end to the rolling termination end, from thin to thick, to be 1:3-75.

Secondly, the present disclosure also provides an artificial tubular tissue with a three-layer structure. The artificial tubular tissue with a three-layer structure includes the following categories.

Specifically, the three-dimensional artificial tubular tissue with a three-layer structure provided by the present disclosure includes a medium-caliber arterial vascular tissue, and a preparation method of a scaffold thereof includes: dividing the scaffold into three different cell seeding regions, and controlling the thickness ratio of the scaffold in the three different regions from the rolling start to the rolling termination end, from thin to thick, to be 1:3-58:3-60 in sequence.

Specifically, the three-dimensional artificial tubular tissue with a three-layer structure provided by the present disclosure also includes a small-caliber arterial vascular tissue, and a preparation method of a scaffold thereof includes: dividing the scaffold into three different cell seeding regions, and controlling the thickness ratio of the scaffold in the three different regions from the rolling start end to the rolling termination end, from thin to thick, to be 1:2-25:3-48 in sequence.

Specifically, the three-dimensional artificial tubular tissue with a three-layer structure provided by the present disclosure also includes a medium-caliber venous vascular tissue, and a preparation method of a scaffold thereof includes: dividing the scaffold into three different cell seeding regions, and controlling a thickness ratio of the scaffold in the three different regions from the rolling start end to the rolling termination end, from thin to thick, to be 1:2-38:3-70 in sequence.

Specifically, the three-dimensional artificial tubular tissue with a three-layer structure provided by the present disclosure also includes a small-caliber venous vascular tissue, and a preparation method of a scaffold thereof includes: dividing the scaffold into three different cell seeding regions, and controlling the thickness ratio of the scaffold in the three different regions from the rolling start end to the rolling termination end, from thin to thick, to be 1:5-50:7-66 in sequence.

**EP 4 692 318 A1**

Specifically, the three-dimensional artificial tubular tissue with a three-layer structure provided by the present disclosure also includes a female-urethral tissue, and a preparation method of a scaffold thereof includes: dividing the scaffold into three different cell seeding regions, and controlling the thickness ratio of the scaffold in the three different regions from the rolling start end to the rolling termination end, from thin to thick, to be 1:5-27:8-60.

Specifically, the three-dimensional artificial tubular tissue with a three-layer structure provided by the present disclosure also includes a male urethral tissue, and a preparation method of a scaffold thereof includes: dividing the scaffold into three different cell seeding regions, and controlling the thickness ratio of the scaffold in the three different regions from the rolling start end to the rolling termination end, from thin to thick, to be 1:4-28:10-66 in sequence.

Specifically, the three-dimensional artificial tubular tissue with a three-layer structure provided by the present disclosure also includes a vas deferens tissue, and a preparation method of the scaffold thereof includes: dividing the scaffold into three different cell seeding regions, and controlling the thickness ratio of the scaffold in the three different regions from the rolling start end to the rolling termination end, from thin to thick, to be 1:4-700:16-1000 in sequence.

In addition, the present disclosure provides a three-dimensional artificial tubular tissue with a four-layer structure. The artificial tubular tissue with a four-layer structure includes the following categories.

Specifically, the three-dimensional artificial tubular tissue with a four-layer structure provided by the present disclosure includes an intestinal tissue, and a preparation method of a scaffold thereof includes: dividing the scaffold into four different cell seeding regions, and controlling the thickness ratio of the scaffold in the four different regions from the rolling start end to the rolling termination end, from thin to thick, to be 1:2-4:2-30:3-35.

Specifically, the intestinal tissue provided by the present disclosure includes small intestine, and protrusions with an axial height of 4-7 mm and a width of 2-3 mm are further provided at a rolling start end region of the small intestine scaffold.

Further, the protrusions include villi and crypts, where the villi have a radial length of 1,200-1,600 $\mu$m and an axial width of 300-600 $\mu$m; and the crypts have a neck diameter of 2-10 $\mu$m, and a lumen diameter of 40-50 $\mu$m.

Specifically, the intestinal tissue provided by the present disclosure also includes large intestine, and crypts are provided at a rolling start end region of the large intestine scaffold, with a distribution density of 100 per square millimeter in an inner layer, a radial depth of 1,500-2,200 $\mu$m, and a diameter of 400-500 $\mu$m.

Specifically, the three-dimensional artificial tubular tissue with a four-layer structure provided by the present disclosure includes an esophageal tissue, and a preparation method of a scaffold thereof includes: dividing the scaffold into four different cell seeding regions, and controlling the thickness ratio of the scaffold in the four different regions from the rolling start end to the rolling termination end, from thin to thick, to be 1:2-100:5-600:20-1000.

Specifically, the three-dimensional artificial tubular tissue with a four-layer structure provided by the present disclosure includes a tracheal tissue, and a preparation method of a scaffold thereof includes: dividing the scaffold into four different cell seeding regions, and controlling the thickness ratio of the scaffold in the four different regions from the rolling start end to the rolling termination end, from thin to thick, to be 1:4-9:15-38:25-56.

Further, a third cell seeding area of the tracheal tissue scaffold from the rolling start end also includes a portion made of a polymer material with a mechanical strength higher than 5-10 MPa or of chondrocytes.

Further, the portion with high mechanical strength is annular and has at least one opening.

Specifically, the three-dimensional artificial tubular tissue provided by the present disclosure includes a fallopian tube tissue, and a preparation method of a scaffold thereof includes: dividing the scaffold into four different cell seeding regions, and controlling the thickness ratio of the scaffold in the four different regions from the rolling start end to the rolling termination end, from thin to thick, to be 1:2-150:3-300:20-1000.

Further, a villus structure is provided in the cell seeding region of the fallopian tube tissue scaffold near the rolling start end. The villus structure is a protrusion with an axial height of 10-11 $\mu$m and a diameter of 0.2-0.3 $\mu$m.

A second objective of the present disclosure is to provide a three-dimensional artificial tubular tissue prepared by any of the above methods.

Specifically, the three-dimensional artificial tubular tissue includes artificial nerves, artificial arterial blood vessels, artificial venous blood vessels, artificial small intestines, artificial large intestines, artificial esophagus, artificial tracheas, artificial urethras, artificial fallopian tubes and artificial vas deferens.

Further, the artificial arterial blood vessels include medium-caliber arterial blood vessels and small-caliber arterial blood vessels.

Further, the artificial venous blood vessels include medium-caliber venous blood vessels and small-caliber venous blood vessels.

Further, the artificial urethra includes male urethra and female urethra.

A third objective of the present disclosure is to provide use of a three-dimensional artificial tubular tissue prepared by any of the above methods, wherein the three-dimensional artificial tubular tissue is used as a replacement for a damaged tissue or as an *in vivo* implant.

The beneficial effects of the present disclosure are as follows.

(1) The present disclosure provides a method for preparing a three-dimensional artificial tubular tissue. By controlling the thickness ratio between different cell seeding regions on the scaffold, the method effectively realizes the design of the three-dimensional topological structure of the scaffold, promotes the three-dimensional growth of cells, and ensures that during the rolling process of the scaffold, each rolled scaffold can provide sufficient tissue thickness for the current cell tissue, such that there will be no cavity between the cell layers of the same tissue, and the cells can better adhere and grow on the same scaffold; meanwhile, the present disclosure can prevent the migration of cells between different layers by arranging the loose layer and the dense layer on the scaffold.

(2) The method for preparing tubular tissue provided by the present disclosure effectively solves the issues of the existing methods, such as the inability to eliminate the cavities between the cell layers, the high tendency for blood leakage and arterial dissection, and the risk of causing aneurysms.

## BRIEF DESCRIPTION OF DRAWINGS

Figure 1 is a schematic diagram of the method for preparing an artificial blood vessel as described in Example 1.

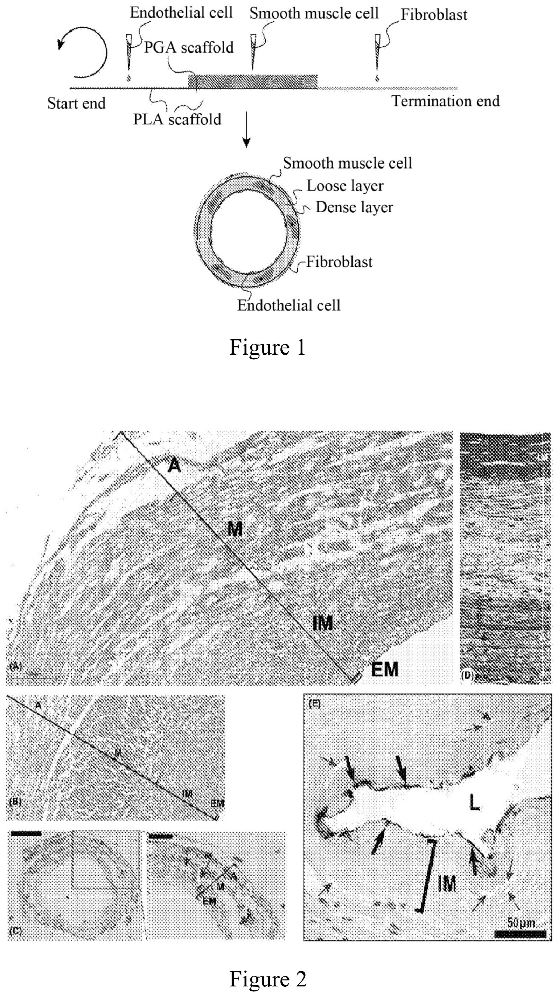

Figure 2 is a characterization diagram showing the tissue structure of an artificial blood vessel; Panel (A) shows that the artificial blood vessel prepared by the method of Example 1 of the present disclosure are decellularized and stained using Masson's trichrome staining, where the blue area represents the extracellular matrix mainly composed of collagen, and the red arrow represents the internal membrane; Panel (B) shows that the decellularized human autologous blood vessel is stained using Masson's trichrome staining, where the blue area represents the extracellular matrix mainly composed of collagen; Panel (C) shows that the artificial blood vessel prepared with a three-layer rolled method reported by Cheng Shiyu and his team in 2017 is stained using Masson's trichrome staining, where the blue area represents the extracellular matrix mainly composed of collagen, and the red arrow points to the cavity formed by the multi-layer rolled method for preparing the blood vessels ; Panel (D) shows that the multi-layered artificial blood vessel reported by Nicolas L'heureux and his team in 1997, is stained using Masson's trichrome staining, where the blue represents the extracellular matrix mainly composed of collagen, the dark red area represents the cell nucleus, and the red arrow points to the cavity formed by the multi-layer rolled method for preparing the blood vessels ; Panel (E) shows LifelineTM, multi-layered artificial blood vessel reported by Marissa Peck and her team in 2011, where the brown area represents immunostaining for CD31, the blue area represents counterstaining for the cell nucleus, the black arrow points to endothelial cells, and the red arrow points to the cavity formed by the multi-layer rolled method for preparing the blood vessels . EM: Endothelial membrane; IM: Internal membrane; M: Media; A: Advertitia.

Figure 3 is a physical picture of an artificial blood vessel prepared by the method of the present disclosure.

Figure 4 shows an axial tensile test of the artificial blood vessel prepared by the method of the present disclosure.

Figure 5 shows a radial ductility test of the artificial blood vessel prepared by the method of the present disclosure.

Figure 6 shows the Young's modulus test results of the artificial blood vessels, the internal mammary arteries, and the artificial blood vessels made of synthetic materials prepared by the method of the present disclosure.

Figure 7 shows the bursting pressure test results of artificial blood vessels prepared by different methods.

Figure 8 shows the suture retention test results of artificial blood vessels prepared by different methods.

Figure 9 shows the compliance test results of artificial blood vessels prepared by different methods.

Figure 10 shows the elongation at break test results of artificial blood vessels prepared by different methods.

Figure 11 shows the long-term patency test results of artificial blood vessels prepared by different methods.

Figure 12 is a cross-sectional photograph of artificial blood vessels prepared by different methods before the start of mechanical stimulation culture.

Figure 13 is a cross-sectional view of the middle inner wall of each artificial blood vessel after two months of mechanical stimulation but before decellularization.

Figure 14 is a diagram showing the evolution of the cavity ratio in three stages, from the initial stage of culture of each artificial blood vessel to the completion of the decellularization process of the blood vessel.

Figure 15 is a diagram showing the evolution of the cavity ratio of the artificial blood vessels prepared in the examples of the present disclosure and the comparative example in three stages, from the initial stage of culture to the completion of the decellularization treatment of the blood vessels.

Figure 16 shows angiographic images of each artificial blood vessel taken three months after implantation.

Figure 17 is an angiographic image of the artificial blood vessel prepared by the methods of Examples 2-7 of the present disclosure taken three months after implantation.

Figure 18 is an angiographic image of the artificial blood vessel prepared by the methods of Comparative Examples 1-9 of the present disclosure taken three month after implantation.

Figure 19 shows a comparison result of the tissue cell density between the scaffold comprising both the dense layer and loose layer structures as described in the present disclosure and the scaffold comprising only the loose layer structure.

Figure 20 shows the statistics on the distribution of tissue cell density in each layer after section staining, comparing the scaffold comprising both the dense layer and loose layer structures with the scaffold comprising only the loose layer structure, following 2 weeks, 4 weeks, and 6 weeks of culture.

Figure 21 is a diagram showing the intestinal crypt and villus structures designed in the artificial intestine of the present disclosure respectively.

Figure 22 is a diagram showing the structure of the prismatic protrusion designed in the artificial nerve of the present disclosure.

Figures 23-30 show the results of cavities in artificial blood vessel tissues described in existing technologies 1 to 7.

## DETAILED DESCRIPTION

In order to make the objectives, technical solutions and advantages of the present disclosure more clearly understood, the present disclosure is described in detail below with reference to the embodiments. It is necessary to point out that the following embodiments are only used to explain and illustrate the present disclosure and are not intended to limit the present disclosure. Some non-essential improvements and adjustments made by those skilled in the art based on the above contents described in the section of Summary still fall within the scope of protection of the present disclosure.

Example 1

A method for preparing a 3 mm caliber artificial blood vessel included the following steps:

(1) preparing a scaffold;

(2) seeding cells on the scaffold; and

(3) rolling into a three-dimensional tubular structure and performing *in vitro* culture.

The cell scaffold used in this 3mm caliber artificial blood vessel product had three cell seeding regions: an endothelial cell seeding region, a smooth muscle cell seeding region, and a fibroblast seeding region. The three regions can be connected by interweaving fibers through needle-punching, using degradable sutures, electrospinning/melt spinning with overlapping spinning, or ultrasonic welding, to form a blood vessel with an inner radius of 1.5 mm and a length of 12 cm. The scaffold thicknesses of the three cell seeding regions on the cell scaffold, from thin to thick, were controlled to be 100 $\mu$m, 400 $\mu$m and 500 $\mu$m in sequence, that is, the thickness ratio of scaffold from thin to thick was 1:4:5 in sequence.

The scaffold in the endothelial cell seeding region had a double-layer structure consisting of a dense layer and a loose layer. The material of the dense layer of the scaffold preferably had a smaller fiber diameter of 8 $\mu$m, a smaller pore diameter of 3 $\mu$m, a thinner scaffold thickness of 5 $\mu$m, and a width of 12.6 mm. A flat and uniform scaffold was preferred, and the scaffold material could be degraded within 4-6 weeks. This dense layer could be prepared by weaving, melt-blowing or electrospinning methods. The material of the loose layer of the scaffold preferably had a fiber diameter of 5 $\mu$m, a larger pore diameter of 30 $\mu$m, and a scaffold thickness of 95 $\mu$m. The dense layer and the loose layer structures were bonded together by means of biological glue or the like. The results of culturing cells on this cell scaffold were as follows: the cell suspension could quickly penetrate into the loose layer, while the cells would not fall through the gaps to the bottom of the seeding vessel during the cell seeding process; endothelial cells in the suspension could quickly aggregate into cell clusters, and the clustered cells could grow adhering to the flat dense layer without migrating to the smooth muscle layer; and the formed endothelial tissue could be smoother, and the decellularized product was more conducive to the differentiation of autologous vascular cells into endothelial cells after implantation.

The smooth muscle cell seeding region also had a double-layer structure, with the dense and loose layers differing from those in the endothelial cell region in terms of parameters, characteristics, and functions. The dense layer preferably had a smaller fiber diameter of 5 $\mu$m, a pore diameter of 3 $\mu$m, a scaffold thickness of 10 $\mu$m, and a width of 13 mm. The preferred scaffold material could degrade within 2-5 weeks. This dense layer could be prepared by weaving, melt-blowing or electrospinning. The loose layer preferably had a fiber diameter of 2 $\mu$m, a larger pore diameter of 10 $\mu$m, and a scaffold thickness of 390 $\mu$m. The scaffold was preferably flat and uniform and could be degraded within 2-5 weeks. The two layers of structure were bonded together by means of biological glue or the like. The results of culturing cells on this cell scaffold were as follows: the smooth muscle cell suspension could quickly penetrate into the loose layer, and the smooth muscle cells in the suspension could quickly aggregate into cell clusters to form smooth muscle tissue; and under axial mechanical stimulation of different intensities, different amounts of elastin and collagen were secreted, which was conducive to the differentiation of autologous vascular cells into smooth muscle cells after implantation.

The scaffold in the fibroblast seeding area was composed of a single-layer loose scaffold. The preferred parameters were a smaller fiber diameter of 2 $\mu$m, a larger pore diameter of 12 $\mu$m, a thicker scaffold thickness of 500 $\mu$m, and a width of 26 mm. The scaffold was preferably flat and uniform, and was made of a scaffold material that could be degraded within 2-5 weeks. The results of culturing cells on this cell scaffold were as follows: the fibroblast suspension could quickly penetrate into the loose layer; the fibroblasts in the suspension could quickly aggregate into cell clusters to form fibroblast tissue, secret different amounts of elastin, fibronectin and collagen, and exchange substances and provide nutrition for vascular tissue during the culture process.

The materials used for the dense layer and loose layer of the scaffold mentioned above could include at least one of PGA, PLA, PCL, PLC, PPDO, PHBV, HA, PU, PET, PP, PE, PA, PMMA, PTFE, PES, PEEK, PS, PP, PMMA, PC, PGA-TMC, PGA-CL, chitosan or collagen, or copolymer materials thereof.

The specific preparation method of the scaffold was listed as follows: PGA-CL, PLGA or PLC was combed into a loose structure through a melt-blowing process, and the same or different materials were dissolved in an organic solvent and then electrospun onto the loose structure under an electric field via an electrospinning process, forming a scaffold structure combining dense and loose. The electric field used was controlled at 10-30 kV, the nozzle diameter was in a range of 0.1-0.8 mm, an extrusion speed of the polymer was controlled between 0.1 and 20 mL/min, a dissolved concentration of the polymer was in a range of 5%-50%, and a distance between the nozzle and the collector was controlled between 2-30 cm. Collectors including rollers and flat plates could be used. Polylactic acid (PLA) or polyhydroxybutyrate (PHB) could also be used as an adhesive, applied between the loose layer and the dense layer at a rate of 3 g/cm$^2$. The layers were then overlapped and left to stand for 20 minutes to 8 hours to allow bonding and fixation. Similarly, thinner scaffolds could also be fused with thicker scaffolds using this method. Alternatively, the overlapping areas of the thinner and thicker scaffolds could be bonded via ultrasonic welding: the polymer material was heated in situ by ultrasonic waves, with one welding spot set every 1-10 cm$^2$ and each welding process lasting 0.1 ms to 1 s, enabling the two layers to bond. Another method involved textile processing of the two layers to be bonded via high-pressure spunlacing. For spunlacing, the nozzle pressure was controlled within 70-180 Pa, the nozzles used were generally in a range of 0.15-0.3 mm, a water flow rate was controlled between 0.2 and 2 liters/minute, and a spunlacing density was set at 2-10/cm$^2$.

After the scaffold was prepared, 200 $\mu$L of cells were seeded per 10 mm$^2$ in the target area through a dispensing process. The cell-seeded scaffold was tightly rolled along a clean stainless steel rod with a radius of 1.5 mm. After rolling, the overlapping multiple layers were sutured and fixed at both ends of the tubular scaffold using surgical sutures and then removed from the metal rod.

The rolled tubular biological tissue was cultured in a culture medium at 37°C for 8 weeks under continuous periodic mechanical pulse stimulation. During this process, the culture medium was replaced every 72 hours. After the culture of blood vessels were completed, the scaffold material has been completely degraded, and the tissue was decellularized and immersed in a preservation solution containing sodium heparin and modified proteins such as endothelial growth factor for more than 24 hours before it could be used for clinical treatment or long-term storage.

The small-caliber artificial blood vessel prepared by the above method had a smooth internal membrane and had radial and axial extensibility that matched that of autologous blood vessels, and strong compliance. It would not easily cause lumen collapse under torsion or bending.

In the above preparation method, a schematic diagram of the operation, including dividing the scaffold into different cell seeding regions, seeding cells in these different regions, and rolling the scaffold into blood vessels from the rolling start end to the rolling termination end after cell seeding, was shown in Figure 1

Example 2

The method of Example 1 was referred to, except that the scaffold thicknesses of the three cell seeding regions on the cell scaffold, from thin to thick, were controlled to be 100 μm, 500 μm and 700 μm in sequence, namely, the scaffold thickness ratio from thin to thick was 1:5:7 in sequence.

Example 3

The method of Example 1 was referred to, except that the scaffold thicknesses of the three cell seeding regions on the cell scaffold, from thin to thick, were controlled to be 100 μm, 1,000 μm and 4,000 μm in sequence, namely, the scaffold thickness ratio from thin to thick was 1:10:40 in sequence.

Example 4

The method of Example 1 was referred to, except that the scaffold thicknesses of the three cell seeding regions on the cell scaffold, from thin to thick, were controlled to be 100 μm, 200 μm and 300 μm in sequence, namely, the scaffold thickness ratio from thin to thick was 1:2:3 in sequence.

Example 5

The method of Example 1 was referred to, except that the scaffold thicknesses of the three cell seeding regions on the cell scaffold, from thin to thick, were controlled to be 100 μm, 200 μm and 210 μm in sequence, namely, the scaffold thickness ratio from thin to thick was 1:2:2.1 in sequence.

Example 6

The method of Example 1 was referred to, except that the scaffold thicknesses of the three cell seeding regions on the cell scaffold, from thin to thick, were controlled to be 100 μm, 2,500 μm and 4,800 μm in sequence, namely, the scaffold thickness ratio from thin to thick was 1:25:48 in sequence.

Example 7

The method of Example 1 was referred to, except that the scaffold thicknesses of the three cell seeding regions on the cell scaffold, from thin to thick, were controlled to be 10 μm, 800 μm and 5,000 μm in sequence, namely, the scaffold thickness ratio from thin to thick was 1:80:500 in sequence.

Example 8

The method of Example 1 was referred to, except that the scaffold thicknesses of the three cell seeding regions on the cell scaffold, from thin to thick, were controlled to be 10 μm, 500 μm and 660 μm in sequence, namely, the scaffold thickness ratio from thin to thick was 1:50:66 in sequence.

Comparative Example 1

The method of Example 1 was referred to, except that the scaffold thicknesses of the three cell seeding regions on the cell scaffold, from thin to thick, were controlled to be 100 μm, 180 μm and 500 μm in sequence, namely, the scaffold thickness ratio from thin to thick was 1:1.8:5 in sequence.

Comparative Example 2

The method of Example 1 was referred to, except that the scaffold thicknesses of the three cell seeding regions on the cell scaffold, from thin to thick, were controlled to be 1 μm, 40 μm and 3,200 μm in sequence, namely, the scaffold thickness ratio from thin to thick was 1:40:3,200 in sequence.

Comparative Example 3

The method of Example 1 was referred to, except that the scaffold thicknesses of the three cell seeding regions on the cell scaffold, from thin to thick, were controlled to be 100 $\mu$m, 150 $\mu$m and 2,000 $\mu$m in sequence, namely, the scaffold thickness ratio from thin to thick was 1:1.5:20 in sequence.

Comparative Example 4

The method of Example 1 was referred to, except that the scaffold thicknesses of the three cell seeding regions on the cell scaffold, from thin to thick, were controlled to be 10 $\mu$m, 12 $\mu$m and 3,000 $\mu$m in sequence, namely, the scaffold thickness ratio from thin to thick was 1:1.2:300 in sequence.

Comparative Example 5

The method of Example 1 was referred to, except that the scaffold thicknesses of the three cell seeding regions on the cell scaffold, from thin to thick, were controlled to be 10 $\mu$m, 180 $\mu$m and 3,100 $\mu$m in sequence, namely, the scaffold thickness ratio from thin to thick was 1:1.8:310 in sequence.

Comparative Example 6

The method of Example 1 was referred to, except that the scaffold thicknesses of the three cell seeding regions on the cell scaffold, from thin to thick, were controlled to be 10 $\mu$m, 820 $\mu$m and 3,000 $\mu$m in sequence, namely, the scaffold thickness ratio from thin to thick was 1:82:300 in sequence.

Comparative Example 7

The method of Example 1 was referred to, except that the scaffold thicknesses of the three cell seeding regions on the cell scaffold, from thin to thick, were controlled to be 10 $\mu$m, 850 $\mu$m and 2,500 $\mu$m in sequence, namely, the scaffold thickness ratio from thin to thick was 1:85:250 in sequence.

Comparative Example 8

The method of Example 1 was referred to, except that the scaffold thicknesses of the three cell seeding regions on the cell scaffold, from thin to thick, were controlled to be 10 $\mu$m, 1,000 $\mu$m and 4,000 $\mu$m in sequence, namely, the scaffold thickness ratio from thin to thick was 1:100:400 in sequence.

Comparative Example 9

The method of Example 1 was referred to, except that the scaffold thicknesses of the three cell seeding regions on the cell scaffold, from thin to thick, were controlled to be 10 $\mu$m, 18 $\mu$m and 4,800 $\mu$m in sequence, namely, the scaffold thickness ratio from thin to thick was 1:1.8:480 in sequence.

Comparative Example 10

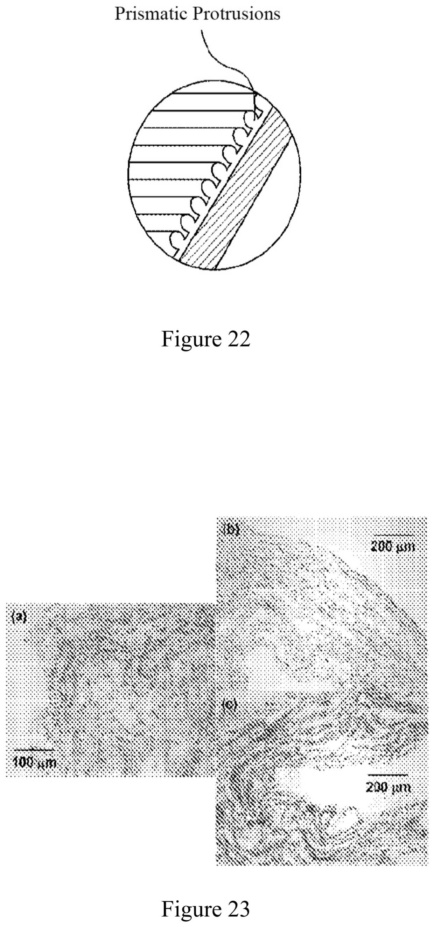

Existing technology I: Artificial blood vessels were prepared by the method described in the literature (Stankus J J, Soletti L, Fujimoto K, et al. Fabrication of cell microintegrated blood vessel constructs through electrohydrodynamic atomization[J]. Biomaterials, 2007, 28(17): 2738-2746). The artificial blood vessel sections described in this literature had cavities (as shown in Figure 23 ). According to the cavity ratio calculation method described in the subsequent part of the present disclosure, the cavity ratio of the blood vessels was 15% after implantation into animals for three months.

Comparative Example 11

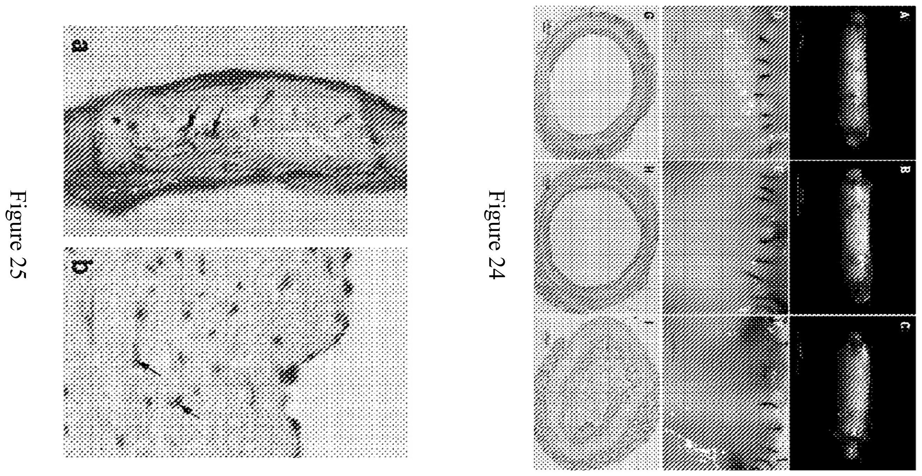

Existing technology II: Artificial blood vessels were prepared according to the method described in the literature (Zheng W, Wang Z, Song L, et al. Endothelialization and patency of RGD-functionalized vascular grafts in a rabbit carotid artery model[J]. Biomaterials, 2012, 33(10): 2880-2891.). The artificial blood vessel sections described in the literature had cavities (as shown in Figure 24), and the cavity ratio of the blood vessels was 12% after implantation into animals for three months.

Comparative Example 12

Existing technology III: Artificial blood vessel were prepared according to the method described in the literature (Teebken O E, Haverich A. Tissue engineering of small diameter vascular grafts[J]. European Journal of Vascular and Endovascular Surgery, 2002, 23(6): 475-485.). The artificial blood vessel sections described in the literature had cavities (as shown in Figure 25), and the cavity ratio of the blood vessels was 10% after implantation into animals for three months.

Comparative Example 13

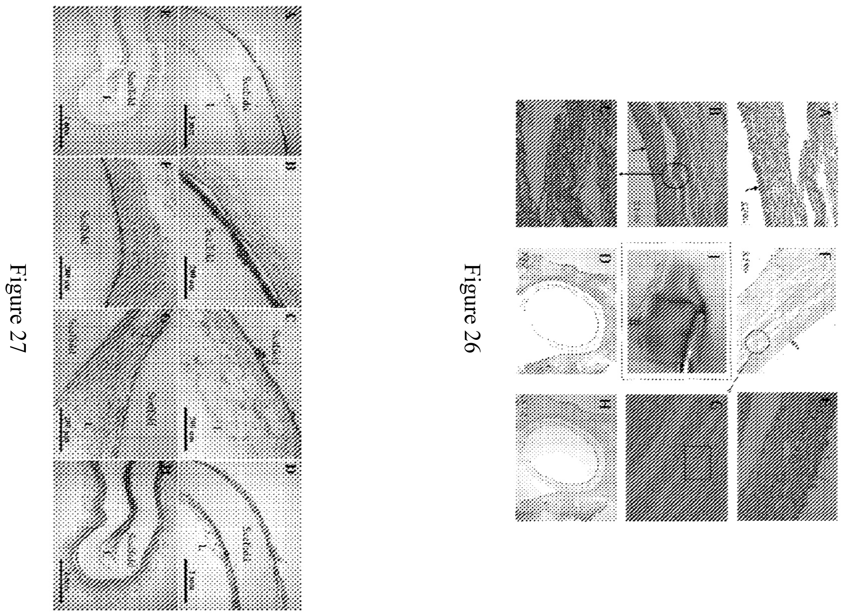

Existing technology IV: Artificial blood vessels were prepared according to the method described in the literature (Kim M J, Kim JH, Yi G, et al. In vitro and in vivo application of PLGA nanofiber for artificial blood vessel[J]. Macromolecular Research, 2008, 16: 345-352.). The artificial blood vessel sections described in the literature had cavities (as shown in Figure 26), and the cavity rate of the blood vessels was 14% after implantation into animals for three months.

Comparative Example 14

Existing technology V: Artificial blood vessels were prepared according to the method in the literature (Wang W, Hu J, He C, et al. Heparinized PLLA/PLCL nanofibrous scaffold for potential engineering of small-diameter blood vessel: Tunable elasticity and anticoagulation property[J]. Journal of Biomedical Materials Research Part A, 2015, 103(5): 1784-1797.). The artificial blood vessel sections described in this literature had cavities (as shown in Figure 27), and the cavity rate of the blood vessels was 13% after implantation into animals for three months.

Comparative Example 15

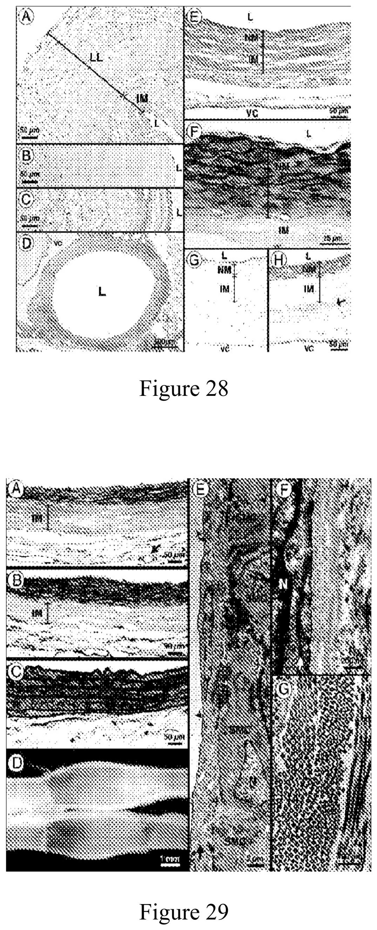

Existing technology VI: Artificial blood vessels were prepared according to the method in the literature (L'Heureux N, Dusserre N, Konig G, et al. Human tissue-engineered blood vessels for adult arterial revascularization[J]. Nature Medicine, 2006, 12(3): 361-365.). The artificial blood vessel sections described in the literature had cavities (as shown in Figures 28 and 29), and the cavity rate of the blood vessels was 10% after implantation into animals for three months.

Comparative Example 16

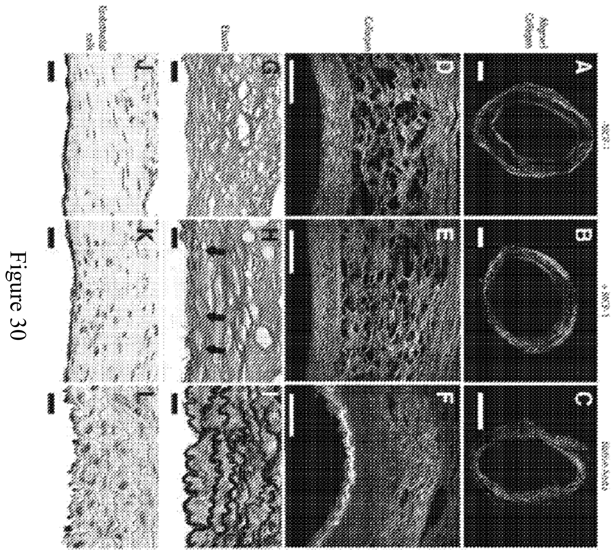

Existing technology VII: Artificial blood vessels were prepared according to the method described in the literature (Talacua H, Smits AIPM, Muylaert DEP, et al. In situ tissue engineering of functionalsmall-diameter blood vessels by host circulating cells only[J]. Tissue Engineering Part A, 2015, 21(19-20): 2583-2594.). The artificial blood vessel sections described in the literature hadcavities (as shown in Figure 30), and the cavity rate of the blood vessels was 9% after implantation into animals for three months.

Experimental Example 1

Taking the 3 mm caliber artificial blood vessel prepared with reference to the method of Example 1 of the present disclosure as an example, after decellularization, a cross section of the blood vessel was taken and the tissue structure was characterized using Masson's trichrome staining.

It can be clearly observed from Figure 2 that the prepared artificial blood vessel has a structure composed of three types of cells, where the endothelial membrane secreted by endothelial cells is denser than the internal membrane and the media made of smooth muscle cells, and has a thickness of only a single cell. Smooth muscle cells form two structures: the internal membrane and the media. The extracellular matrix of the internal membrane is distributed in a circumferential direction, while the extracellular matrix of the media is distributed along the axial direction. The adventitia is composed of the extracellular matrix of fibroblasts and has the loosest structure compared to the other three layers. All of the above characteristics are highly consistent with human autologous blood vessels. The artificial blood vessel prepared by the present disclosure has a dense internal membrane similar to that of the autologous blood vessel.

Experimental Example 2

The key to the mechanical indicators of artificial blood vessels lies in whether their mechanical properties can match those of human autologous blood vessels, while having better toughness and being able to maintain functionality for a longer time. For example, the nonlinear elastic constant of the small-caliber arterial blood vessels mentioned in the present disclosure can cause the blood vessel to have a greater stress when the inner diameter of the blood vessels increases due to a sudden increase in the blood flow, thereby preventing the blood vessels from further expansion. Meanwhile, under normal pulsation, blood vessels can expand and contract freely, reducing the pressure inside the blood vessels. In contrast, an artificial blood vessel made of a single material will not be able to match the autologous blood vessel at the anastomosis site due to its linear elastic curve, accelerating intimal hyperplasia and thrombosis.

In this experiment, the following mechanical properties were tested on tissue-engineered artificial blood vessels (6 cm in length, 3 mm inner diameter, N = 6), synthetic blood vessels (3 mm inner diameter, PU, N = 5), and decellularized human internal mammary arteries (about 2.8 mm, N = 7).

(I) Circumferential/Longitudinal Tensile Test:

In this experiment, a vascular tissue was stretched along its cross section until it broke by was using a uniaxial tensile device, and then recorded and estimated its maximum tensile strength and elongation at break.

First, a 5 mm-long segment of the blood vessel was cut and placed on two parallel hooks made of 1.2 mm-diameter stainless steel wires. The hooks were then pulled apart at a speed of 35 mm/min until the tissue broke, with data sampled using a LabView data acquisition system.

In addition, a 20 mm-long blood vessel was cut and the two ends were fixed on two 60 mm-long stainless steel wire clamps. The clamps were then pulled apart at a speed of 45 mm/min until the tissue broke, with data sampled using a LabView data acquisition system.

(II) Suture Retention Test:

For the s suture retention test, a blood vessel segment approximately 15 mm in length was cannulated onto a vertical metal support shaft and connected to an electronic scale. A simple interrupted suture with a 2 mm interval between stitches was performed using a BV-1 needle with 5-0 suture thread. The suture was pulled out at a constant speed of 120 mm/min until the tissue broken, with data sampled using a LabView data acquisition system.

(III) Bursting Pressure Test:

A blood vessel of approximately 5 cm in length was cannulated at both ends and pressurized with phosphate-buffered saline (PBS) at a rate of 80 to 100 mmHg/s until the tissue ruptured. Data was sampled using a LabView data acquisition system and an electronic pressure gauge.

(IV) Compliance Test:

Compliance testing required measuring the ratio of the difference in the inner diameter of the blood vessel in a resting state and under pressurized conditions to the pressure difference. A 5-cm long vessel segment was tensioned to 0.460 Newtons and pressurized with phosphate-buffered saline (PBS) until 50 to 300 mmHg was reached, and highdefinition images were taken for measurement of the outer diameter. Prior to testing, cross-sections from the same vessel were fixed in formalin to obtain the geometry at rest from the histological sections.

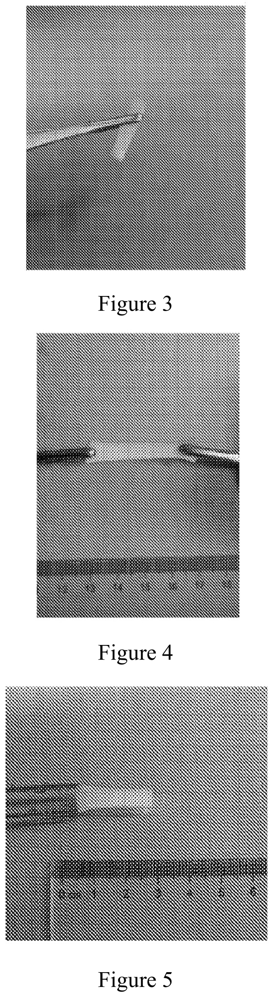

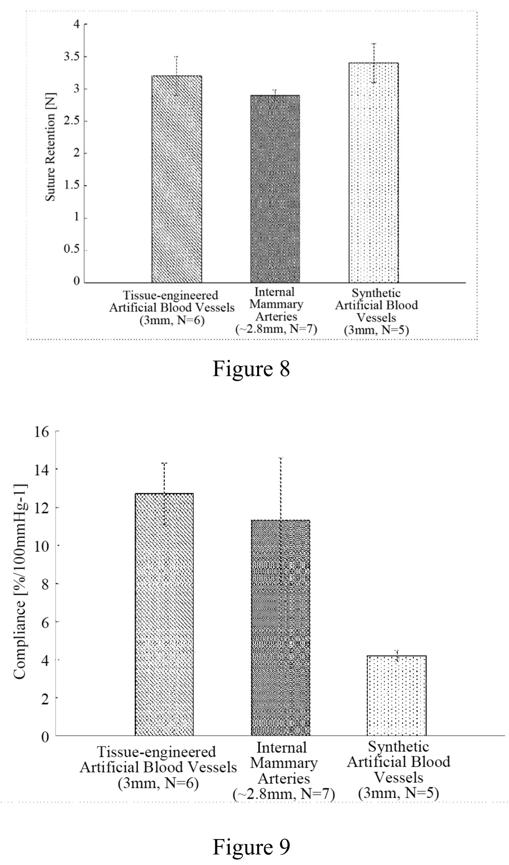

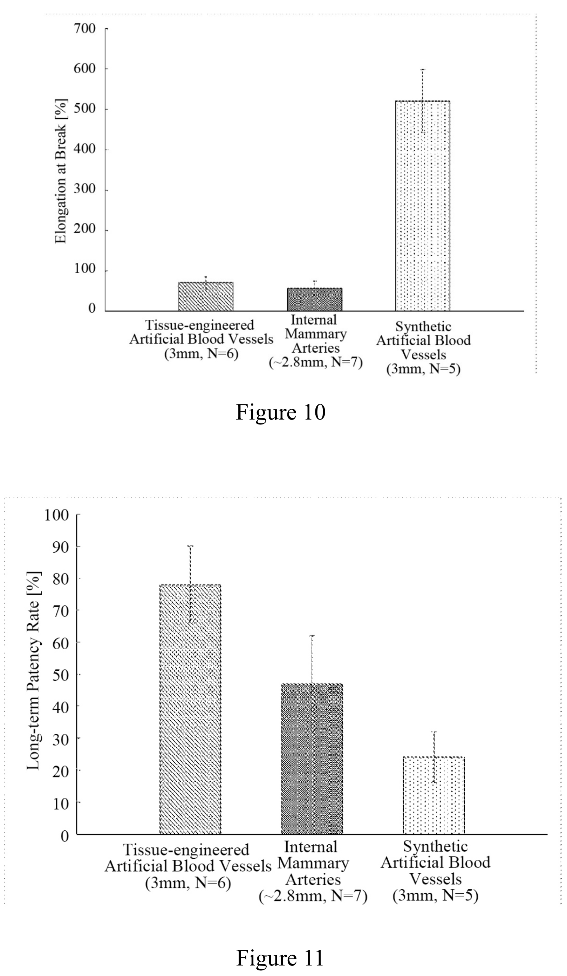

The experimental results are shown in Figures 3 to 11.

Figure 3 shows an artificial blood vessel prepared by the method of Example 1 of the present disclosure. The artificial blood vessel is found to be soft and smooth, and the tissue grows evenly. Figure 4 shows that both ends of the artificial blood vessel are stretched using forceps, and it is found that the artificial blood vessel can be stretched to about twice its own length, indicating that the artificial blood vessel prepared by the patented method has excellent axial stretchability. Figure 5 shows that the inner diameter of an artificial blood vessel is stretched using forceps, and it is found that the artificial blood vessel can be stretched to about 1.5 times its own inner diameter, indicating that the artificial blood vessel prepared by the method of the present disclosure has excellent radial ductility.

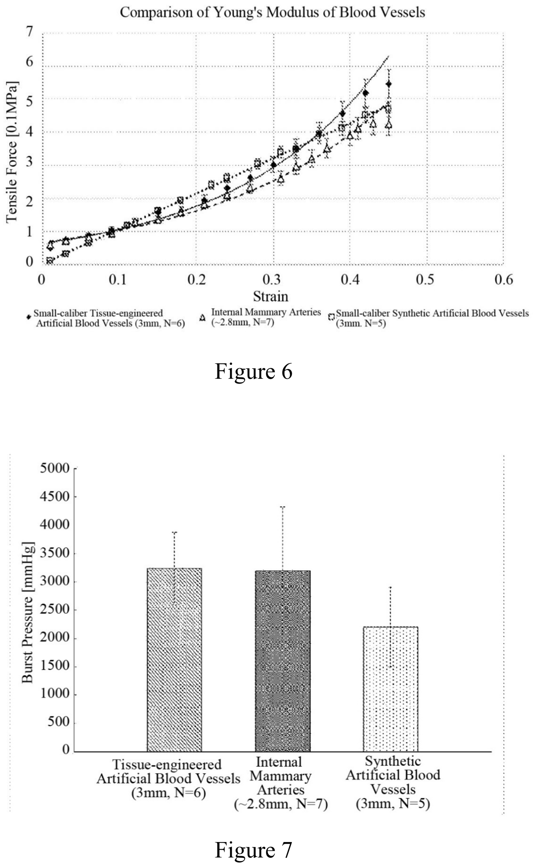

From the comparison of mechanical properties in Figure 6 to Figure 11, it can be seen that the mechanical properties of the artificial blood vessel prepared by the present disclosure are highly similar to those of the autologous blood vessel.

Further research reveals that the tissue engineered artificial blood vessels prepared by the method of the present disclosure have no cavities in their internal membranes. However, multi-layer blood vessels prepared by other rolling methods in the existing technologies, such as those prepared by using multi-layer rolling and stacking within the same region, can create multiple cavities of varying sizes within the region. The presence of these cavities significantly reduces the mechanical strength in the circumferential direction and causes difficulties in the self-wound repair of blood vessels after implantation, greatly increasing the risk of post-operative endothelial hyperplasia, smooth muscle cell migration, and aneurysm formation.

In addition, the artificial blood vessels prepared by the method of the present disclosure can match the upstream and downstream autologous blood vessels after implantation in terms of their Young's modulus and compliance, which is far superior to other existing synthetic material blood vessels. Meanwhile, the blood vessels prepared by the method of the present disclosure have better performance in long-term patency, are less likely to bend during implantation, and have matched hemodynamics at the suture site, further reducing the risk of endothelial hyperplasia and improving long-term patency (see Experimental Example 3 for details).

Experimental Example 3

Testing of Cavity Rate in Tissue-Engineered Artificial Blood Vessels

According to the report in the literature (DOI: https://doi.org/10.7461/jcen.2020.22.3.127, Clinical analysis of young adult patients with ruptured intracranial aneurysms: a single-center study of 113 consecutive patients. J Cerebrovasc Endovasc Neurosurg. 2020; 22(3): 127-133. Published online September 21, 2020), the mean size of intracranial aneurysms in young adult patients (<=40 years old) is in a range of $6.6 \pm 3.7$ mm. However, in the young adult patients, aneurysms smaller than 5mm can cause symptoms. The study points out that young adult patients have a higher rupture risk of small aneurysm rupture than middle-aged and elderly patients. In the study, 94.2% of ruptured aneurysm in young adult patients are smaller than 10 mm, and 47.6% are smaller than 5 mm.

When an aneurysm is initially formed, the thickness of the blood vessel wall that constitutes the cavity has not yet reached the mechanical strength to support itself. At this stage, if it is frequently exposed to high pressure and high-intensity living environment, it is highly likely to trigger aneurysm rupture. Not only the size of the aneurysm but also its shape has a great impact on the chance of rupture. The literature points out that aneurysms with long or other irregular shapes are more likely to rupture.

From the above results, it can be concluded that if there are cavities between the walls of the artificial blood vessel itself, especially gaps near the inner wall, the endothelial cell layer will easily rupture under the impact of blood flow after decellularization treatment. This rupture causes blood to flow into the smooth muscle layer, leading to the formation of aneurysms. Therefore, cavities in finished artificial blood vessels should be kept to a minimum or eliminated. Cavities and gaps of any shape and size will pose hidden dangers to patients. Taking healthy blood vessels as the reference, the inventors statistically analyzed a total of 300 stained sections of human radial arteries and found that the cavity rate was $2 \pm 0.6\%$. Therefore, in this experiment, the cavity rate of the artificial blood vessel wall was set to be at least less than 2%.

The aneurysm mechanism described above also suitable to other artificial tubular tissues, such as intestinal tissues or urethral tissues. Cavities in the interlayer of the tube wall can also cause digestive juices, gas or urine to flow into the middle tissue layer, forming saccular aneurysms. It can also rupture under pressure and threaten the patient's life. Therefore, there should be no cavities in any sense and no gaps in any form inside the tube wall of the artificial tubular tissues.

For example, when there are cavities in vascular implants, such cavities can cause different forms of vascular lesions and eventually lead to necrosis and blockage. Among these lesions, the most common one is hemangioma. When the blood vessel wall surrounding the cavity ruptures, blood will flow into the cavity, tearing the blood vessel wall and causing the cavity to expand, forming a false lumen. The expanded false lumen wall will block blood from flowing in the correct direction, leading to blood reflux and blood vessel blockage.

On the other hand, when autologous cells attempt to repair the cavity, it may also cause thrombosis. Once blood flow seeps into the blood vessel wall and flows into the cavity, platelets and cholesterol accumulate here to form thrombi. The formation of thrombi will further reduce the mechanical strength and flexibility of blood vessels, making them more susceptible to rupture. The continued accumulation of thrombi will make the blood vessel lumen narrower, hindering the normal flow of blood, resulting in a decrease in flow velocity or even blockage. When thrombi dislodge, free-floating blood clots in the blood vessel can also cause blockage of downstream blood vessels.

Meanwhile, the cavity will reduce the strength of the blood vessel itself, lower its burst strength, and make vascular endothelia rupture more likely to occur. Smooth muscle cells in the vascular media will migrate to the inner wall of the blood vessels, causing endothelial hyperplasia, which in turn narrows the inner diameter of the blood vessels and results in blockage.

In addition to the above-mentioned lesions, cavities are more likely to accumulate infectious agents and cause inflammation, which in turn causes connective tissue hyperplasia, leading to vascular stenosis and blockage. In short, cavities inside the blood vessels may cause a variety of vascular lesions, including hemangiomas, thrombosis, vascular endothelial rupture, and endothelial hyperplasia, and ultimately lead to vascular blockage. The above-mentioned lesions are also the primary cause of early-stage blockage in artificial blood vessels requiring interventional treatment.

Under this premise, the inventors of this patent conducted research on all prepared previously artificial tubular tissues and counted the number and size of the cavities. The preparation methods of artificial blood vessel samples refer to the following methods, respectively: (a) constructing multi-layer tubular blood vessels by traditional multi-layer rolling and stacking method (A completely biological tissue-engineered human blood vessel, 1998, NICOLASL'HEUREUX et al.); (b) constructing three-layered tubular blood vessels by using a self-rolling method (CN 108653815 B, National Center for Nanoscience and Technology); and (c) preparation of tissue-engineered artificial blood vessels by using the method of the present disclosure.

Compared with the artificial blood vessels prepared by the above-mentioned methods (a) and (b), the artificial blood vessels prepared by the method (c) of the present disclosure can minimize the formation of cavities while ensuring the mechanical properties and physiological structure of the blood vessels by controlling the thickness between layers. This ensures that the prepared blood vessels will not develop the above-mentioned lesions due to cavities, greatly improving the patency of vascular implants and achieving excellent results in three-month animal experiments.

(1) The experimental method is as follows:

Blood vessels with an inner diameter of 4 mm and a length of 12 cm were prepared according to the method of Example 1, with 30 samples for each type. Samples were collected in four stages. In the first stage, five vessels were removed for cross-sectional imaging after cell seeding and rolling and before the start of mechanical stimulation culture. In the second and third stages, ten blood vessels were collected before and after decellularization after eight weeks of culture, respectively. In the fourth stage, a 5 cm segment was cut from each of five blood vessels, which were then implanted into New Zealand rabbits for radial artery replacement surgery, and the patency of the implanted vessels was observed after three months.

In the first stage, because the connection between layers within the vessel wall had not yet been established, the detachment between layers was likely to occur during sectioning. Additionally, the cavities at this stage were visible to the naked eye. Therefore, only cross-sectional imaging was used to evaluate the cavities within the blood vessel wall. During evaluation, samples were taken from both ends (3 cm from each end) and the middle (6 cm from either end) of each blood vessel to observe the uniformity of interlayer fusion. After processing, the blood vessels in the second, third and fourth stages were sent to professional institutions for sectioning and staining. Samples were taken from each blood vessel at both ends (3 cm from each end) and the middle (6 cm from either end) to observe the uniformity of interlayer fusion. The specific sectioning and staining procedures refer to publicly available standard methods and would not be explained in detail here. However, in order to reduce the impact of sectioning on the vascular wall structure, the following treatments were performed on the blood vessels during sample preparation:

1) taking two blood vessels with a total length of 12 cm and immersing them in a petri dish containing PBS buffer to clean off the residual culture medium in the blood vessels;

2) selecting the exact middle section of the 12 cm blood vessel and cutting off the vessel at 3 cm and 9 cm from one end towards the other end using medical scissors;

3) trimming irregularities on the cross-section of the blood vessels by using a disposable scalpel, wherein rotary cutting was performed on the irregular areas, starting cutting from the outer edge of the cross-section; throughout the cutting process, the blade was kept in contact with both the vessel wall being cut and the backing plate to avoid radial compression of the vessel;

4) naming the two processed blood vessels as (a) and (b), respectively, where vessel (a) was decellularized, and vessel (b) was used as a blank control group;

5) placing blood vessels (a) and (b) into separate centrifuge tubes containing paraformaldehyde fixative, allowing the paraformaldehyde solution to submerge the blood vessels, and then sending them for examination.

Since blood vessels prepared using different preparation methods exhibit different inner diameters, the following formula was used to define the cavity rate to enable a more objective quantitative comparison of the cavities between layers for different blood vessels,:

$$\text{Cavity Rate (\%)} = (\text{Cavity Area/Inner Lumen Area}) \times 100\%.$$

The cavity area refers to the sum of all spaces larger than the cell diameter in photographs or section images.

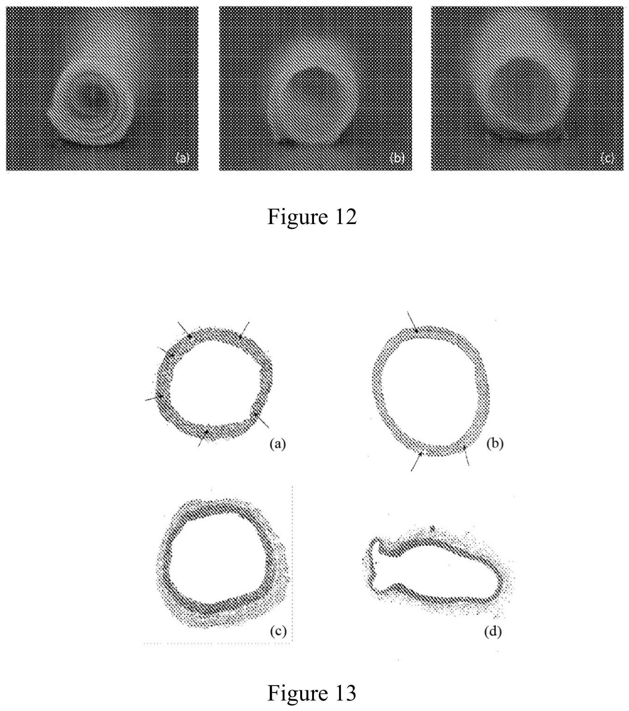

(2) Experimental results: the experimental results are shown in Figures 12-15.

Figure 12 shows cross-sectional photographs of artificial blood vessels before the start of mechanical stimulation culture, wherein: Panel (a) shows the artificial blood vessels prepared by the traditional multi-layer rolling and stacking method; Panel (b) shows the artificial blood vessels prepared by the self-rolling method; Panel (c) shows the artificial blood vessels prepared by the method of the present disclosure. As can be seen from Figure 12, the artificial blood vessels prepared by the traditional multi-layer rolling and stacking method have many cavities between layers before the start of mechanical stimulation culture. The reason is that although biological glue was used for fixation, the multi-layer material itself would always undergo a certain degree of collapse due to gravity, resulting in cavities. For the artificial blood vessels prepared by the self-rolling method, the cavity rate was improved to a certain extent before culture due to the tension of the material itself, however, the uniform thickness of the material made it impossible to achieve a thickness ratio compatible with autologous blood vessels after rolling. However, the blood vessels prepared by the method of the present disclosure could naturally form a suitable thickness ratio after forming, and would not have increased cavities due to collapse. In the early stage of culture, it can realize interlayer adhesion, thereby promoting the fusion and molding of the blood vessel wall.

Figure 13 shows cross-sectional images of the inner wall of the middle segment of blood vessels after two months of mechanical stimulation and before decellularization treatment, wherein: Panel (a) shows the artificial blood vessels prepared by the traditional multi-layer rolling and stacking method; Panel (b) shows the artificial blood vessels prepared by the self-rolling method; Panel (c) shows the artificial blood vessels prepared by the method of the present disclosure; and Panel (d) shows the cross-sectional image of a rat arterial blood vessel, with arrows indicating the larger cavities. As can be seen from the Figure 13, the blood vessels prepared by the traditional multi-layer rolling and stacking method have small and numerous cavities, most of which are located near the endothelial layer. This greatly reduces the strength of the endothelial layer; the cavities have no fixed shape but generally have a large radial distance. For the blood vessels prepared by the self-rolling method, cavities appear at the interfaces between layers. These cavities are mostly long and narrow in shape, and their distance from the endothelial layer is determined by the self-rolling material. However, the blood vessels prepared by the method of the present disclosure have smooth inner walls and a dense structure with no obvious cavities. The inner wall surface of the blood vessels is uniformly covered with a layer of vascular endothelial cells. There are significant differences in density between the outermost fibroblast layer and smooth muscle cells, making the blood vessels more similar to autologous blood vessels. Moreover, the polymeric biodegradable material used as the scaffold have been completely absorbed.

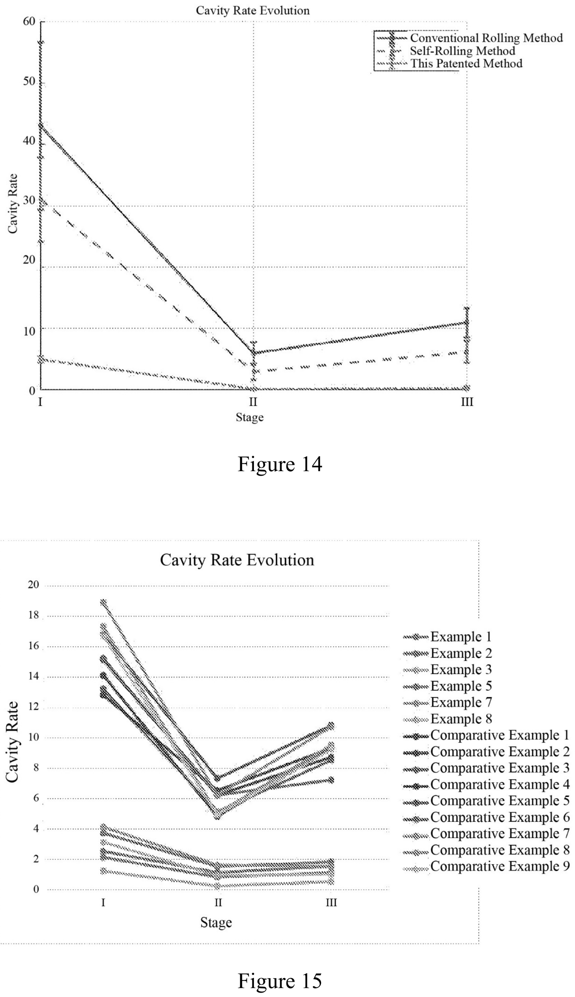

Figure 14 shows the evolution of the cavity rate across three stages, from the early stage of vascular culture to the completion of vascular decellularization treatment. As can be seen from Figure 14, the blood vessels prepared by the traditional multi-layer rolling and stacking method and the self-rolling method have a cavity rate of nearly 40% in the first stage. After mechanical stimulation culture, the biodegradable scaffold contracts, and the tissue layers begin to fuse, resulting in a reduction in cavities. In the third stage after the cell elution treatment, cells that have not fully formed tissues are stripped away, forming new cavities, thus causing the cavity rate to rise. In contrast, the cavity rate of blood vessels prepared by the method of the present disclosure always remain at a low level, and there is no rebound in the cavity rate even after decellularization treatment, indicating that dense tissue have been formed inside the blood vessel wall.

The artificial blood vessels prepared in the examples of the present disclosure and comparative examples were cultured, and their cavity rates were counted in three stages. The results were shown in Table 1, and a cavity rate evolution diagram was plotted. Figure 15 shows the evolution of the cavity rate of different artificial blood vessel samples prepared in the examples and comparative examples across three stages from the early stage of blood vessel culture to the completion of the decellularization treatment of the blood vessels. As can be seen from Figure 15, at the early stage of blood vessel culture, the artificial blood vessels obtained by the method of the examples of the present disclosure have a lower cavity rate, while those obtained by the methods of comparative examples have a higher cavity rate. After the second stage of mechanical stimulation culture, the scaffolds degrade and contract, and the tissue layers begin to fuse, resulting in a decrease in the cavity rate of all samples. In the third stage after the cell elution treatment, the cells that have not fully formed tissues in the comparative examples are stripped away, forming new cavities, thus causing the cavity rate to rise. In contrast, the cavity rate of the artificial blood vessels prepared by the method provided in the examples of the present disclosure always remain at a low level, and there is no rebound in the cavity rate after decellularization treatment, indicating that the dense tissue have been formed inside the blood vessel wall.

Table 1

| Cavity rate (%) | Stage | | |
|---|---|---|---|
| Sample | I | II | III |
| Example 1 | 2.1 | 0.8 | 1.1 |
| Example 2 | 2.5 | 1.1 | 1.5 |

(continued)

| Cavity rate (%) | Stage | | |
|---|---|---|---|
| Sample | I | II | III |
| Example 3 | 1.2 | 0.2 | 0.5 |
| Example 5 | 3.7 | 1.5 | 1.8 |
| Example 7 | 4.1 | 1.6 | 1.5 |
| Example 8 | 3.1 | 0.9 | 1.0 |
| Comparative Example 1 | 15.2 | 6.2 | 8.7 |
| Comparative Example 2 | 14.1 | 4.8 | 9.3 |
| Comparative Example 3 | 13.2 | 5.1 | 8.5 |
| Comparative Example 4 | 12.8 | 6.5 | 9.3 |
| Comparative Example 5 | 16.8 | 7.3 | 10.8 |
| Comparative Example 6 | 15.1 | 6.2 | 7.2 |
| Comparative Example 7 | 17.3 | 5.0 | 9.5 |
| Comparative Example 8 | 18.9 | 6.2 | 10.7 |
| Comparative Example 9 | 16.7 | 4.9 | 9.2 |

To further compare the impact of the cavity rate of the artificial blood vessels prepared in the first stage on the subsequent culture stages, the inventors took the artificial blood vessels prepared by the self-rolling method as an example. By applying degradable biofusion agents mainly including biogelatin, PEG, or fibrin on each layer before rolling, testing showed that the cavity rate of the artificial blood vessels in the first stage was 3%, and then the second and third stage experiments were carried out. The results showed that the cavity rate still increased after culture. After adopting the above treatment method, the cavity rate in the second stage was 2%, but after the third stage, the cavity rate rebounded to more than 6%, indicating that even if the existing preparation processes obtained a good initial cavity rate, they still tended to generate a large number of cavities in the subsequent culture processes.

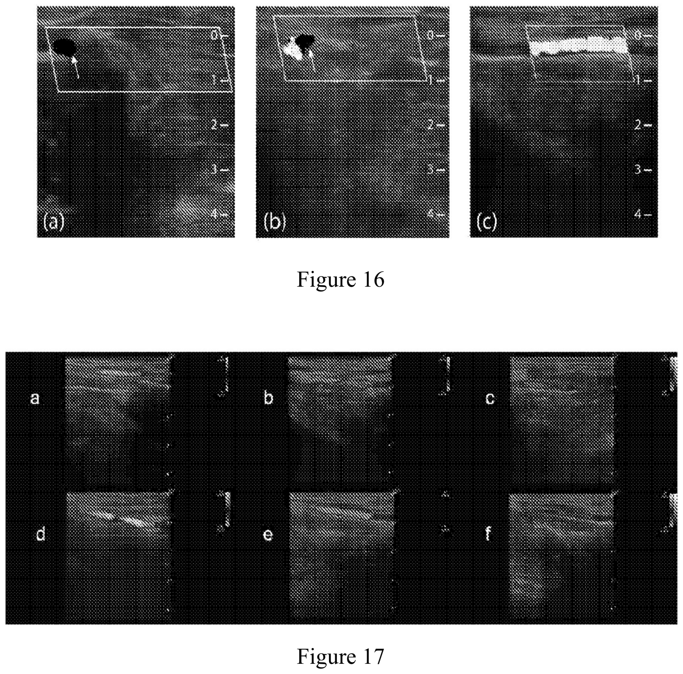

Figure 16 compares the angiographic images of the prepared blood vessels after implantation into animals for three months, wherein, Panel (a) shows the artificial blood vessels prepared by the traditional multi-layer rolling and stacking method; Panel (b) shows the artificial blood vessels prepared by the self-rolling method; and Panel (c) shows the artificial blood vessels prepared by the method of the present disclosure, where the black areas indicated by the arrows are regions with reverse flow. It can be clearly seen from the Figure 16 that the blood vessels prepared by the traditional multi-layer rolling and stacking method are completely occluded and even exhibit reverse flow, with a high possibility of aneurysm formation. The blood flow velocity of the blood vessels prepared by the self-rolling method is significantly slowed down, and thrombus may occur in the blood vessels. Only the blood vessels prepared by the method of the present disclosure maintain at a consistent blood flow velocity with that at the initial implantation, proving that the artificial blood vessels prepared by this patented method have no cavities, and no thrombus or aneurysm occurr.

In this experiment, the artificial blood vessels prepared in Examples 2-7 of the present disclosure were respectively implanted into animals and angiographic observation was performed three months later. The obtained angiographic images are shown in Figure 17, where Panel a to Panel f correspond to the artificial blood vessels prepared by the methods of Examples 2-7, respectively. As can be seen from Figure 17, the artificial blood vessels prepared by the methods provided in the above Examples of the present disclosure maintain unobstructed blood flow throughout, with no occurrence of thrombus or aneurysm.

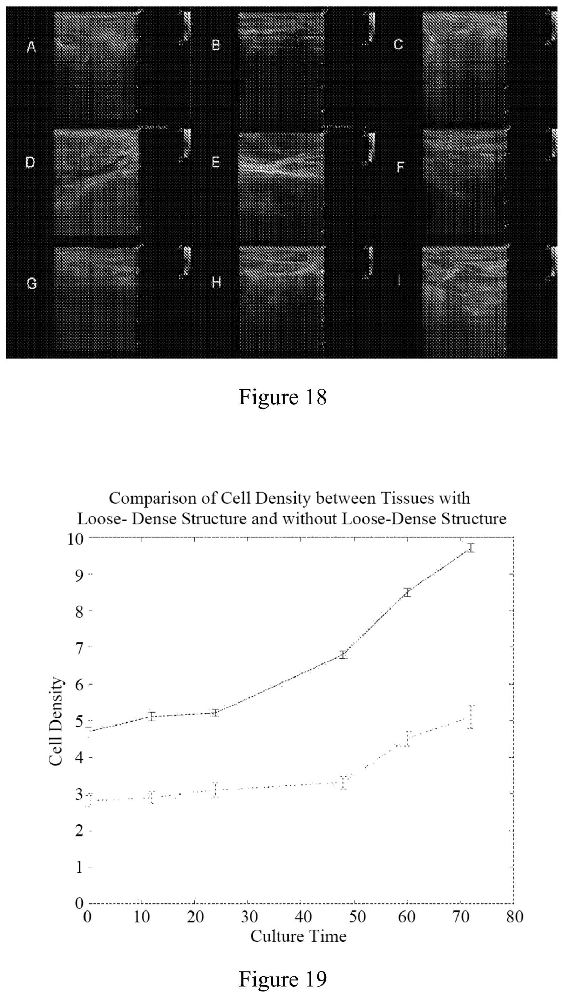

In contrast, the artificial blood vessels prepared in Comparative Examples 1-9 of the present disclosure were implanted into animals, and angiographic observations was performed three months later. The obtained angiographic images are shown in Figure 18, where Panel A to Panel I corresponded to the artificial blood vessel samples prepared by the methods of Comparative Examples 1-9, respectively. As can be seen from Figure 18, the artificial blood vessels prepared by the method provided in the comparative examples all show blood flow obstruction three months after implantation into animals, with a high probability of thrombus or aneurysm.

Experimental Example 4

Comparison was made between the scaffold comprising both the loose layer and dense layer used in the method of the present disclosure and the scaffold comprising only the loose layer. The comparison method was as follows:

(I) Ten loose scaffolds and ten loose-dense composite scaffolds were taken respectively, and fluorescently transfected cells were seeded at specific sites (50,000 cells/cm$^2$), and cultured for 2 days. Every 12 hours, the distribution density and quantity of cells on the scaffolds were counted by a confocal microscope. The results are shown in Figure 19, where the solid line represents the loose-dense composite scaffold, and the dashed line represents the scaffold comprising only the loose structure. The y-axis represents the distribution density of cells (10,000 cells/cm$^2$), and the x-axis represents time. As shown in Figure 19, the cell differentiation rate of the scaffold comprising only the loose layer is lower than that of the loose -dense composite scaffold. Additionally, the cells on the composite scaffold have a narrower distribution range when compared among scaffolds of the same type, making them more suitable for process standardization in mass production.

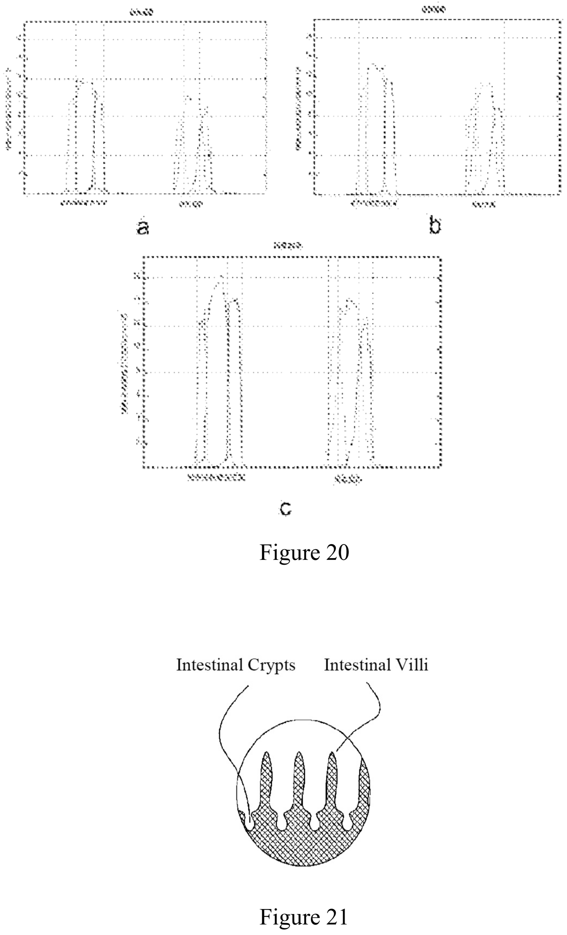

(II) 12 loose tubular scaffolds and 12 loose-dense composite scaffolds seeded with cells were taken respectively. At 2 weeks, 4 weeks, and 6 weeks after culture, 4 scaffolds from each group were sacrificed at each stage. After sectioning and staining, the distribution positions of tissue cells in each layer were counted. The results are shown in Figure 20, where the dashed line represents the loose scaffold, and the solid line represents the composite scaffold. Black indicates the fibroblast layer, dark gray indicates the smooth muscle cell layer, and light gray indicates the endothelial cell layer.

As shown in Figure 20, the distribution boundaries between layers of the tubular tissue prepared using the composite scaffold are more obvious, without excessive overlapping parts. Around 4 weeks, when part of the dense scaffold layer degrades, a small number of cells migrate. Meanwhile, the tubular tissue prepared using the scaffold comprising only a loose structure have unclear cell distribution boundaries, and more cells migrate, making it difficult for the same type of cells to aggregate and grow into clusters to form tissue. As can be seen from the Figure 20, the cell density of the single-type scaffold at 6 weeks of culture is not significantly different from that at 4 weeks. Moreover, smooth muscle cells entering the endothelial layer may increase the chance of endothelial hyperplasia after implantation, posing potential risks for patients.

Example 9

This example provided a method for preparing a venous-arterial fistula for artificial dialysis. The preparation method was different from that of Example 1 only in that the blood vessel diameter was increased from 3 cm to 6 cm, so the width of each region was also appropriately increased to adapt to the inner diameter. The scaffold material of the venous-arterial fistula for artificial dialysis may be PCL, PLGA, PLC or PLA. The cells seeded on the scaffold included human endothelial cells, smooth muscle cells and fibroblasts. The preparation method of the scaffold was the same as that in Example 1. The thickness ratio of the seeded regions of the three cell layers on the scaffold, was controlled from thin to thick to be 1:30:500.

Example 10

A method for preparing a venous-arterial fistula for artificial dialysis was provided with reference to Example 9, except that a thickness ratio of the seeded regions of the three cell layers on the scaffold was controlled from thin to thick to be 1:2:4.

Example 11

This example provided a method for preparing a venous-arterial fistula for artificial dialysis. The difference between the preparation method of Example 11 and that of Example 1 was that: the diameter of the blood vessel was increased from 3 cm to 6 cm, and the venous-arterial fistula only had a two-layer structure including a smooth muscle layer and a fibroblast layer, so the width of each region was also appropriately increased to adapt to the inner diameter. The scaffold material of the venous-arterial fistula for artificial dialysis may be PGLA, PLC or PLA. The cells seeded on the scaffold were human endothelial cells and smooth muscle cells. The preparation method of the scaffold was the same as that in Example 1. The thickness ratio of the seeded regions of the two cell layers on the scaffold was controlled from thin to thick to be 1:20.

Example 12

A method for preparing a venous-arterial fistula for artificial dialysis was provided with reference to Example 11, except that the thickness ratio of the seeded regions of the two cell layers on the scaffold was controlled from thin to thick to be 1:2.

Example 13

A method for preparing a venous-arterial fistula for artificial dialysis was provided with reference to Example 11, except that the thickness ratio of the seeded regions of the two cell layers on the scaffold was controlled from thin to thick to be 1:80.

Example 14

A method for preparing a venous-arterial fistula for artificial dialysis was provided with reference to Example 11, except that the thickness ratio of the seeded regions of the two cell layers on the scaffold was controlled from thin to thick to be 1:40.

Example 15

This example provided a method for preparing an artificial intestinal organ. The difference between the preparation method of Example 15and that of Example 1 was that: the scaffolds for the stem cell region of the intestinal inner wall were made from a hydrogel containing arginine-glycine-aspartic acid. Intestinal stem cells were embedded in hydrogels, and the stem cells and hydrogels were mixed together through suspension culture such that they were evenly distributed in the gel. The hydrogel matrix was u degraded and softened by irradiating with 405 nm light, thereby controlling the spatiotemporal specificity of the gel. The growth and differentiation of stem cells in the gel could be controlled by controlling the location and duration of light exposure. The thickness ratio of the artificial intestinal scaffold was controlled from thin to thick to be 1:2:10:35. Each gel was patterned into *in vivo* crypt dimensions of 20 $\mu$m $\times$ 300 $\mu$m by microlithography and placed in a differentiation medium. The stem cells in the hydrogel were cultured. Differentiation and crypt-villus patterning were induced in the differentiation medium (containing EGF, Noggin, and R-spondin), allowing stem cells to differentiate into different cell types in the gel and form crypt-villus structures. The scaffold material for the artificial intestinal tissue could be PPDO, PHBV, HA or PLC. The cells seeded on the scaffold were intestinal stem cells and smooth muscle cells. The preparation method for the scaffold included the method in Example 1. The following method could also be used: the patterns of intestinal crypts and villi were etched onto a wafer using microlithography transfer. This technology comprised first using microlithography to etch the patterns onto the wafer through photoresist and a photolithography machine, and then transferring the patterns to a polydimethylsiloxane (PDMS) mold. Next, the mold was inverted over the hydrogel layer, and the three-dimensional structure of the villi and crypts was transferred to the scaffold through extrusion and temperature curing. This technology could accurately replicate the morphology of intestinal villi and crypts on the surface of the scaffold, thereby simulating the microstructure of the intestine. The intestinal tissue was also designed with a microscopic topological structure composed of intestinal villi and intestinal crypts. The radial length of the villi was 1200 to 1600 $\mu$m, the axial width was 300 to 600 $\mu$m; the diameter of the crypt neck was 2 to 10 $\mu$m, and the lumen diameter was approximately 40 to 50 $\mu$m (the structure was shown in Figure 21).

Example 16

A method for preparing an artificial intestinal organ was provided with reference to Example 15, except that the thickness ratio of the artificial intestinal scaffold was controlled from thin to thick to be 1:2:500:2,000.

Example 17

A method for preparing an artificial intestinal organ was provided with reference to Example 15, except that the thickness ratio of the artificial intestinal scaffold was controlled from thin to thick to be 1:50:300:1,000.

Example 18

A method for preparing an artificial intestinal organ was provided with reference to Example 15, except that the thickness ratio of the artificial intestinal scaffold was controlled from thin to thick to be 1:80:700:3,000.

Example 19

This example provided a method for preparing an artificial nerve. The preparation method thereof was different from that of Example 1 only in that the thickness ratio of the artificial nerve scaffold from thin to thick was 1:50. In the loose scaffold of the nerve at the rolling start end, there were axially distributed prismatic protrusions, the distribution density of the prismatic protrusions in the inner layer was 10-15 per millimeter, with a length of 5-20 millimeters. Each prismatic protrusion was independent of each other, and was separated from each other by a dense layer of 1-8 microns. The material used for the artificial nerve scaffold could be HA, PLGA, PDO or PLC. The cells seeded in different cell seeding regions of the scaffold were nerve cells, neuroglial cells and Schwann cells respectively. The scaffold preparation methods could be: using microlithography transfer technology, a wafer with a prismatic protrusion structure was first prepared, and then this three-dimensional structure was transferred onto a hydrogel scaffold; and subsequently, the scaffold was rolled along the short side of the prismatic structure such that its long side was parallel to the axis of the tubular tissue. The regions between the prismatic protrusions were subjected to low-temperature stamping for shrinkage with low-temperature stamping technology. The stamping mold comprised patterns interlaced with the prismatic protrusions. During the low-temperature stamping, the temperature was 30 to 180 degrees Celsius, the pressure was 20 to 100 kPa, and the stamping time was 10 to 120 minutes. In addition, in the loose scaffold region of the nerve at the rolling start end, there were axially distributed prismatic protrusions. The distribution density of the prismatic protrusions in the inner layer was 10-15 per millimeter, and the length was 5-20 millimeter. Each prismatic protrusion was independent of each other and was separated from each other by a dense layer of 1-8 microns (the structure was shown in Figure 22).

Example 20

This example provided a method for preparing an artificial trachea. The artificial trachea product had a diameter of 1.2-2.0 cm, a length of 5-10 cm, and a tracheal wall thickness of approximately 1.5 mm, with longitudinal extensibility and lateral rigidity. The trachea had four layers: a tracheal endothelial cell layer, a smooth muscle cell layer, a chondrocyte layer, and a fibroblast layer, where the thickness ratio of each layer from thin to thick was 1:9:38:56.

The difference between the preparation method of Example 20 and that of Example 1 was that the chondrocyte cell layer had a composite structure, which was formed by a dense-loose layer with a thickness of 0.7 mm, and was endowed with a polymer corrugated support structure with high mechanical strength. The polymer material was preferably poly-$\varepsilon$-caprolactone (PCL), which was processed by fused deposition modeling (FDM) 3D printing or electrospinning to form trapezoidal columns with a narrow top and wide bottom. The upper bottom of the trapezoidal column was 0.9 mm, and the angle between the side edge and the vertical direction was $35° \pm 1.5°$. The length accounted for 80% of the dense-loose layer, and the columns were arranged centrally at intervals of 3 mm and connected by ultrasonic welding. Chondrocytes were cultured in a high-cell-density conformation with the supplementation of transforming growth factor $\beta$ (TGF-$\beta$). Under long-term mechanical stimulation, cartilage structures were formed, and type II collagen (COL2), aggrecan (ACAN), and cartilage extracellular matrix (ECM) were secreted. After rolling, a C-shaped cartilage ring scaffold was prepared. The cartilage rings enabled longitudinal stretching and bending: the inner region was compressed, while the outer region beared tension, with a tensile equilibrium modulus ranging from 1 to 20 MPa to adapt to diaphragm contraction, lung expansion, and neck movement. Meanwhile, the C-shaped ring structure allowed the migration of smooth muscle cells and fibroblasts into the gap regions, providing radial and axial ductility matching that of the autologous trachea, and enabling the growth and infiltration of autologous microvascular tissue.

Example 21

This example provided a method for preparing an artificial digestive tract with reference to Example 1. The artificial digestive tract in this example was described in detail below:

1. Small Intestine

The artificial small intestine had an inner diameter of 15 mm to 30 mm, with a four-layer structure: an inner layer, a basement layer, a middle layer and outer layer. The relationship between the wall thickness (t) and the inner diameter (d) was that: t was approximately equal to d/7. The preparation method of the artificial small intestine scaffold was as follows: the scaffold was divided into four different cell seeding regions, and the thickness ratio of the scaffold in the four different regions from the rolling start end to the rolling termination end was controlled to be 1:2:25:35.

The small intestine had a relatively complex inner-layer topological structure, which facilitated the differentiation and metabolism of intestinal stem cells after seeding. The topological structure could be specifically classified into microscopic topological structure and macroscopic topological structure. The microscopic topology was composed of intestinal villi and intestinal crypts. The radial length of the villi was 1200 to 1600 $\mu$m, the axial width was 300 to 600 $\mu$m, the diameter of the crypt neck was 2 to 10 $\mu$m, and the lumen diameter was approximately 40 to 50 $\mu$m. The macrostructure was composed of axial folds similar to the natural structure of Kerckring's folds, which could slow down the movement of food in the intestine. The structure was a protrusion in an axial direction with a height of 4 to 7 mm and a width of 2 to 3 mm.

These two types of topological structures could be prepared by different methods.

2. Large Intestine

The artificial large intestine had an inner diameter of 40 mm to 50 mm. This tissue had a four-layer structure: an inner layer, a basement layer, a middle layer, and an outer layer. The relationship between the wall thickness (t) and the inner diameter (d) was that: t was approximately equal to d/15 to d/7. The preparation method of the artificial large intestine scaffold was as follows: the scaffold was divided into four different cell seeding regions, and the thickness ratio of the scaffold in the four different regions from the rolling start end to the rolling termination end was controlled to be 1:4:30:35.

The large intestine also had a relatively complex inner layer topological structure, mainly composed of intestinal crypts. The distribution density of the crypts in the inner layer was approximately 100 per square millimeter, with a radial depth of 1,500 to 2,200 $\mu$m and a diameter of 400 to 500 $\mu$m. This topological structure could be prepared by different methods.

Example 22

This example provided a method for preparing an artificial esophagus, with reference to Example 1. The artificial esophagus in this example was described in detail below:
The artificial esophagus had an inner diameter of 18 to 22 mm. This tissue had a four-layer structure: an inner layer, a basement layer, a middle layer, and an outer layer. The relationship between the wall thickness (t) and inner diameter (d) was that: t was approximately equal to d/4. The preparation method of the artificial esophagus scaffold was as follows: the scaffold was divided into four different cell seeding regions, and the thickness ratio of the scaffold in the four different regions from the rolling start end to the rolling termination end was controlled to be 1:2:5:20.

The inner wall of the artificial esophagus did not have a special topological structure and had a smooth surface, which was conducive to the differentiation of autologous vascular stem cells into vascular endothelial cells.

Example 23

This example provided a method for preparing an artificial trachea, with reference to Example 1. The artificial trachea in the present example was described in detail below:
The artificial trachea had an inner diameter of 10-23 mm. This trachea had a four-layer structure: an inner layer, a basement layer, a middle layer, and an outer layer. Meanwhile, the relationship between the wall thickness (t) and inner diameter (d) was that: t was approximately equal to d/6, with a total wall thickness of approximately 3000 $\mu$m. The preparation method of the artificial trachea scaffold was as follows: the scaffold was divided into four different cell seeding regions, and thickness ratio of the scaffold in the four different regions from the rolling start end to the rolling termination end was controlled to be 1:9:15:26.

It was noteworthy that the middle layer of the artificial trachea was composed of two parts, one quarter of the circumference was a loose polymer scaffold, and the other three quarters was made of a polymer material with high mechanical strength. This polymer material was characterized by its ability to withstand an axial stress of 5 to 10 MPa without breaking. This section of material was used to simulate the hyaline cartilage of the autologous trachea. Under long-term mechanical stimulation, the seeded chondrocytes secreted type II collagen and chondroitin sulfate, which promoted the differentiation of autologous stem cells into chondrocytes after implantation.

The inner wall of the artificial trachea did not have a special topological structure and featured a smooth surface, which was conducive to the differentiation of autologous stem cells into tracheal mucosal cells. However, the highstrength areas in the middle layer were distributed in a ring shape along the axial direction, with each spacing being 3-9 mm and each ring width being about 7-9 mm.

Example 24

The preparation of an artificial male urethra included the following steps:

(1) preparing a scaffold;

(2) seeding cells on the scaffold; and

(3) rolling into a three-dimensional tubular structure and conducting *in vitro* culture.

The scaffold material and preparation process were in accordance with the method mentioned in Example 1.

The artificial male urethra in this example had a diameter of 5-7 mm. This urethra had a three-layer structure: an inner layer, a middle layer and an outer layer. Meanwhile, the relationship between the wall thickness (t) and diameter (d) was that: t was approximately equal to d/1.2, with a urethral wall thickness of approximately 2-2.6 mm. The preparation method of the artificial male urethra scaffold was as follows: the scaffold was divided into three different cell seeding regions, and the thickness ratio of the scaffold in the three different regions from the rolling start end to the rolling termination end was controlled to be 1:4:10.

It was noteworthy that the middle layer of the artificial trachea was made of a loose elastic polymer material. The inner wall of the artificial male urethra did not have any special topological structure, and had a smooth surface.

Example 25

The preparation of an artificial female urethra included the following steps:

(1) preparing a scaffold;

(2) seeding cells on the scaffold; and

(3) rolling into a three-dimensional tubular structure and conducting *in vitro* culture.

The scaffold material and preparation process were in accordance with the method mentioned in Example 1.

The artificial female urethra in this example had a diameter of 5-7 mm. This urethra had a three-layer structure: an inner layer, a middle layer and an outer layer. Meanwhile, the relationship between the wall thickness (t) and diameter (d) was that: t was approximately equal to d/1.5, with a urethral wall thickness of approximately 2-2.6 $\mu$m. The preparation method of the artificial female urethra scaffold was as follows: the scaffold was divided into three different cell seeding regions, and the thickness ratio of the scaffold in the three different regions from the rolling start end to the rolling termination end was controlled to be 1:27:60.

The inner wall of the artificial female urethra did not have any special topological structure, and had a smooth surface.

Example 26

The preparation of an artificial fallopian tube included the following steps:

(1) preparing a scaffold;

(2) seeding cells on the scaffold; and

(3) rolling into a three-dimensional tubular structure and conducting *in vitro* culture.

The scaffold material and preparation process were in accordance with the method mentioned in Example 1.

The artificial fallopian tube in this example had an inner diameter of 5 to 12 mm. This tissue had a four-layer structure: an inner layer, a basement layer, a middle layer, and an outer layer. The relationship between the wall thickness (t) and inner diameter (d) was that: t was approximately equal to d/6. The preparation method of the artificial fallopian tube scaffold was as follows: the scaffold was divided into four different cell seeding regions, and the thickness ratio of the scaffold in the four different regions from the rolling start end to the rolling termination end was controlled to be 1:2:3:20.

The fallopian tube had a relatively complex inner layer topology, which facilitated the differentiation of stem cells into mucosal cells after seeding. A villus structure was provided in the cell-seeding region of the fallopian tube tissue scaffold near the rolling start end. These villus structures were protrusions in an axial direction with a height of 10 to 11 $\mu$m and a diameter of 0.2-0.3 $\mu$m.

Example 27

The preparation of artificial vas deferens included the following steps:

(1) preparing a scaffold;

(2) seeding cells on the scaffold; and

(3) rolling into a three-dimensional tubular structure and conducting *in vitro* culture.

The scaffold material and preparation process were in accordance with the method mentioned in Example 1.

The artificial vas deferens in this example had an inner diameter of 5 to 7 mm. This tissue had a three-layer structure: an inner layer, a middle layer, and an outer layer. The relationship between the wall thickness (t) and inner diameter (d) was that: t was approximately equal to d/6. The preparation method of the artificial vas deferens scaffold was as follows: the scaffold was divided into three different cell seeding regions, and the thickness ratio of the scaffold in the three different regions from the rolling start end to the rolling termination end was controlled to be 1:4:16.

The vas deferens had a relatively complex inner topological structure, which facilitated the differentiation of stem cells into mucosal cells after seeding. The inner wall of the artificial vas deferens did have any special topological structure, and had a smooth surface.

Example 28

The preparation of artificial neural tissue included the following steps:

(1) preparing a scaffold;

(2) seeding cells on the scaffold; and

(3) rolling into a three-dimensional tubular structure and conducting *in vitro* culture.

The scaffold material and preparation process were in accordance with the method mentioned in Example 1.

This artificial neural tissue in this example had a two-layer structure including an inner layer and an outer layer. The preparation method of the artificial neural tissue scaffold was as follows: the scaffold was divided into two different cell seeding regions, and the thickness ratio of the scaffold in the two different regions from the rolling start end to the rolling termination end was controlled to be was controlled to be 1:20.

Several two-layer nerves of the aforementioned artificial neural tissue could be bundled into a bundle. The bundled artificial nerve was wrapped by two layers of outer sheath tissue, with the thickness ratio of the scaffold in the two regions from the rolling start end to the rolling termination end controlled to be 1:12.

This artificial nerve in this example had a relatively complex topological structure. In the loose scaffold region at the rolling start end of the nerve, there were axially distributed prismatic protrusions. The distribution density of the prismatic protrusions in the inner layer was 10-15 per millimeter, with a length of 5-20 millimeters. Each prismatic protrusion was independent of each other and was separated from each other by a dense layer of 1-8 microns.

Example 29

Three-Dimensional Patterning of Cell Seeding of Vascular Tissue

(1) The tubular vascular tissue was usually prepared as a felt-type structure, with a large porosity and a certain elasticity of the pores, allowing to rebound and repair the pores after needle insertion. Meanwhile, since the tubular vascular tissue needs to be fixed horizontally on the cell seeding instrument during operation, its structure had a certain rigidity. When preparing such a felt-type scaffold, the existing felt cloth made of highly biocompatible degradable or non-degradable materials was first used to form a tubular scaffold by rolling and then the fibers at the seams were cross-fused by needle punching. The thickness of the scaffold could be controlled by the number of rolled layers, ranging from 100 $\mu$m to 500 $\mu$m.

(2) Both ends of the scaffold tube were connected to a sleeve made of a highly biocompatible non-degradable elastic materials, and their seams were tightly sutured with surgical sutures. The outermost two ends were connected to a set of male parts of Luer quick connectors and fixed to these connectors with sutures. This section of the scaffold could then be docked and fixed with the cell seeding device through a quick-connect method.

(3) When starting the culture, it was necessary to ensure that the liquids in all necessary feed bottles had a sufficient remaining volume. Meanwhile, the cell suspension and PBS should be kept at 37 degrees Celsius, which was suitable for preserving their growth activity. The needles were kept in the cleaning tank and constantly immersed in 75% alcohol before use.

(4) Endothelial Cell Seeding: before seeding, the stirring device in the endothelial cell suspension vessel was turned on. This step was to make the cells evenly suspended in the liquid to avoid uneven distribution of cells squeezed out by the needle during cell seeding. First, the alcohol from the cleaning tank was drained, and 100-150 mL of PBS was directly infused from the Feed Bottle with 4 ports (Feed Bottle 4) which was controlled by the fluid path system by using an air pump to the needle for cleaning the pipeline. It should be noted that the pipeline needed to be cleaned, sterilized or replaced regularly. The cleaning here is to flush out and remove the alcohol from the needle to avoid residual alcohol killing the cells to be seeded. Then, the Feed Bottle 4 was closed via a solenoid valve, and the Feed Bottle 1 was opened, and the endothelial cells were transferred to the needle through the air pump until the liquid discharged from the needle changed from colorless to the rose red color of the culture medium. At this point, the air pump pressure was maintained at 0.5 to 1 kPa slightly above the standard atmospheric pressure, such that the liquid in the catheter was maintained in a state where it would not be squeezed out by external forces. At this point, the preparation work was completed.

(5) The needle was moved to a position 2-3 mm away from the leftmost end of the tubular scaffold by controlling Lead Screw 1 and Screw Rod 1. A needle was then inserted into the inner wall area of the scaffold to seed the endothelial cells. The position of the inner wall was determined by the varying thickness of the scaffold wall and the thickness occupied by the required cells. For example, for an existing blood vessel with an inner diameter of 6 mm and a thickness of 500 $\mu$m, the endothelial cells needed to attach to a layer of the inner wall. Herein, the inner wall was defined as the position 450 $\mu$m from the outermost layer. The needle was inserted to a depth of 450 $\mu$m into the scaffold wall via the distance between the sensor and Screw Rod 2 and Lead Screw 2, where 1-5 $\mu$m of endothelial cell suspension was extruded using piezoelectric material. Subsequently, the needle was then lifted up, and the hydrophobic needle ensured that the seeded droplets of cell suspension remained at the seeding location. The needle was moved 300-500 $\mu$m to the right and the same operation was started until the needle reached a position 2 mm away from the rightmost end of the tubular scaffold. After seeding this row of cells, the tubular stent was rotated by 2 degrees via Servo Motor 1 to begin the next round of endothelial cell seeding.

(6) After endothelial cells had been seeded on the entire inner wall of the scaffold (and the scaffold had been rotated one full circle), the needle was moved to the cleaning tank and rinsed with ultrapure water. Solenoid Pinch Valve 1 was closed, and Solenoid Pinch Valve 4 was opened; the pipeline was cleaned with PBS from Feed Bottle 4. The stirring of endothelial cells in Feed Bottle 1 was stopped, and the stirring of smooth muscle cells in Feed Bottle 2 was started. Solenoid Pinch Valve 4 was closed, and Solenoid Pinch Valve 2 was opened. The smooth muscle cell suspension was directed toward the needle via the air pump until the color of the outflowing liquid changed from colorless to the rose red of the cell culture medium. At this point, the air pump pressure was maintained at 0.5 to 1 kPa slightly higher than the standard atmospheric pressure, such that the liquid in the catheter was maintained in a state where it would not be squeezed out by external forces.

(7) The needle was moved via Lead Screw 1 and Screw Rod 2 1 to a position 5 mm away from the suture joint of the left sleeve. It then dropped downward 15-20 $\mu$L of cell suspension 1 mm above the sleeve using the air pump, after which it moved 1 mm to the right and dropped the cell suspension again-this was repeated until the needle reached a position 2-3 mm to the right of the sleeve. The purpose of this step was to allow smooth muscle cells to penetrate the suture joint between the sleeve and the scaffold, enabling vascular cells to form a dense cell community at the joint after initial culture. This prevented leakage of the scaffold in the later culture stage or breakage caused by scaffold degradation. Similarly, the same operation was performed on the sleeve on the right side. Smooth muscle cells were mainly distributed in the middle layer of blood vessels to provide good mechanical properties. Therefore, the needle was inserted into the 300 $\mu$m position of the scaffold wall under the guidance of Lead Screw 2 and Screw Rod 2. The smooth muscle cell suspension was then infused into the scaffold via the air pump, with each injection spaced at intervals of 500-1000 $\mu$m. After the scaffold was rotated one full circle, the seeding of the next type of cell was carried out.

(8) Following the same cell fluid replacement steps, the fibroblast suspension was injected with a needle at a depth of 100 $\mu$m from the outermost layer of the scaffold wall. Using the method mentioned above, 10-15 $\mu$L of fibroblasts were injected until the entire surface of the scaffold was covered with fibroblasts. With this, cell seeding was completed. The needle was retracted into the cleaning tank for cleaning and storage, in preparation for the next cell seeding.

(9) It should be noted that two fluid ejection methods for cell seeding had been mentioned in this example. When small-volume fluid ejection ($\mu$L level) was required, piezoelectric materials were used for driving. When larger volumes of fluid (10-100 $\mu$L) were needed, the air pump was directly controlled for fluid regulation.

(10) The seeded tissue was removed from the female connector on the device and proceeded to the next step of culture.

Example 30

Three-Dimensional Patterning of Cell Seeding of Intestinal Tissue

(1) The difference between this example and the vascular three-dimensional cell seeding described in Example 10 was that the endothelial surface of the intestine required preferred topological structures, such as villi and concavities, and the inner diameter of the intestine was larger than that of the blood vessels (greater than 10 mm). In view of these two characteristics, the method of seeding cells in a planar manner followed by three-dimensional construction was preferred.

(2) The specific differences from Example 10 included different cell types and distribution orders. The specific order of distribution was intestinal endothelial cells, fibroblasts, and smooth muscle cells. For example, an intestinal tract with a length of 12 cm, an inner diameter of 10 mm and a wall thickness of 500 $\mu$m required a planar scaffold with a length of 140 mm, containing 32 mm of intestinal endothelial cells, 33 mm of fibroblasts, and the remaining portion as smooth muscle cells.

(3) Another specific difference was that the topological structure described above needed to be constructed using a gel-type material (such as hydrogel), and its specific construction method was as follows. A PDMS mold that had the same size as the area required for the hydrogel was placed on a planar scaffold. Using the example above, it was a square frame with dimensions of 32 mm $\times$ 100 mm, a height of 5 mm, and a wall thickness of 4 mm. The hydrogel was injected firstly into this mold with a cell seeding machine. The the scaffold comprising the mold and hydrogel was then transported to the three-dimensional structure patterning area by the conveyor belt, where contained a PDMS stamp made by replicating a photolithographic silicon wafer. Under the action of lead screw and screw rod, the stamp descended and pressed the aforementioned villi and crypts structures onto the hydrogel, while the hydrogel was heated to complete curing. After curing was completed, the mechanical claws around the stamp peeled the PDMS mold off the scaffold and discarded it together with the stamp. The scaffold was then transported back to the cell seeding area to complete cell seeding similar to that in Example 11, as well as tissue rolling similar to that in Example 11. At this point, cell seeding of the artificial intestine was completed, and it could proceed to the next culture stage.

(4) A major advantage of using this rolling method was that fibroblasts easily migrated to other cell layers and interfered with intestinal endothelial cells, thereby affecting the function of intestinal tissue. By rolling the degradable scaffold, the scaffold itself could act as a physical barrier in the early stage of culture, thereby ensuring that the intestinal function remained unaffected after culture completion.

Example 31

Three-Dimensional Patterning of Cell Seeding of Artificial Muscle Tissue

(1) To generate dense muscle cells to simulate the texture of muscle, this example performed three-dimensional planarization with rounded-edge prismatic structures on the planar scaffold before rolling, such that the gaps between the prisms formed tubular cavities to achieve the exchange of substances inside and outside the cells during culture.

(2) The process of this specific example was similar to that described in Example 11, which included adding a mold to the membrane-type scaffold to perfuse the hydrogel, three-dimensionally patterning the hydrogel, and then seeding the cells. The difference was that after cell seeding, tight three-dimensional rolling was performed. The difference from Example 11 was that there was only one lumen in the middle that was negligible compared to the tissue. Meanwhile, only one type of bovine skeletal muscle cells was used in this example. For example, to form a muscle tissue 1.5 cm in thickness and 10 cm in length, a planar scaffold of 36 cm $\times$ 11 cm in length and width was required. A PDMS mold with an internal length of 35 cm, width of 10 cm, height of 5 mm, and wall thickness of 5 mm was used on this planar. A different PDMS stamp was then used. The specific difference was that unlike the stamp that simulated the inner wall of the intestine, this stamp could form rounded-edge prismatic protrusions with a height of 500 $\mu$m and a spacing of 300

$\mu$m. After completing cell seeding inside the hydrogel scaffold, the entire planar scaffold was transported to the rollingss device for rolling with an inner diameter of 0.5 mm, as described in Example 10. The resulting tissue was as shown in the Figure. The rolled tissue could proceed to the next culture stage.

(3) A major advantage of the 3D muscle tissue constructed using this rolling method was that it could form capillary channels around the muscle tissue for substance exchange, thereby improving the overall survival rate of dense muscle tissue during culture.

Example 32

Three-Dimensional Patterning and Cell Seeding of Skin Tissue

The specific method referred to Example 1. The skin itself did not need to be rolled as a planar tissue. Its three-dimensional structure was specifically reflected in the different cells used in each layer of the structure. Therefore, compared with performing cell patterning in different regions of the skin scaffold, this example dispensed different cells at different depths in the same region to achieve the purpose of three-dimensional patterning. For example, a piece of skin with a length and width of 10 cm $\times$ 10 cm and a thickness of 500 $\mu$m required a membranous scaffold of 11 cm $\times$ 11 cm and a thickness of 200 $\mu$m and the hydrogel was perfused in the 10 cm $\times$ 10 cm region of the scaffold using the hydrogel formation method described in Example 13. The difference was that only a hydrogel layer with a thickness of 300 $\mu$m was required to be formed here, without three-dimensional patterning. After the hydrogel layer was formed, different cells were seeded at different depths: fibroblasts at 250 $\mu$m, dermal cells at 150 $\mu$m, and epidermal cells at 100 $\mu$m. Another difference was that compared to suturing elastic non-degradable materials at both ends of the scaffold, the elastic non-degradable materials were sutured around the periphery of the skin tissue scaffold here, and smooth muscle cells were used to reinforce the suture joints. After cell seeding was completed, the cell scaffold could proceed to the next stage of culture.

In the above embodiments, as long as the scaffold thickness ratio design is within the scope of protection of the present disclosure, the cavity between the layers can be eliminated well, such that the cavity rate of the artificial tissue material is less than 2% after implantation for three months. However, the scaffold thickness ratio design that is not within the scope of protection of the present disclosure cannot eliminate the cavity between the layers well. Even if the initial cavity rate is lower than 5%, the cavity rate in the subsequent third stage will rise back to 8%-15%. The inventors will not list them one by one here.

## Claims

1. A method for preparing a three-dimensional artificial tubular tissue, comprising the following steps:

   (1) preparing a rollable scaffold;
   (2) seeding cells on the scaffold;
   (3) rolling a product obtained in step (2) into a three-dimensional tubular structure and conducting *in vitro* culture to obtain the three-dimensional artificial tubular tissue;
   wherein a method for preparing the rollable scaffold comprises any one of the following methods:

      Method 1: dividing the rollable scaffold into two different cell seeding regions in sequence from a rolling start end to a rolling termination end, controlling widths of the two different cell seeding regions along a rolling direction of the scaffold such that each of the different cell seeding regions forms at least one complete tubular layer after rolling, and the tubular layer located on an outer layer is capable of wrapping the tubular layer located on an inner layer after rolling; and controlling a thickness ratio of the scaffold in the two different cell seeding regions from thin to thick to be 1:2-80;
      Method 2: dividing the rollable scaffold into three different cell seeding regions in sequence from a rolling start end to a rolling termination end, controlling widths of the three different cell seeding regions along a roll direction of the scaffold such that each of the different cell seeding regions forms at least one complete tubular layer after rolling, and the tubular layer located on an outer layer is capable of sequentially wrapping the tubular layer located on an inner layer after rolling, and controlling a thickness ratio of the scaffold in the three different cell seeding regions from thin to thick to be 1:2-80:2-3000; or
      Method 3: dividing the rollable scaffold into four different cell seeding regions in sequence from a rolling start end to a rolling termination end, controlling widths of the four different cell seeding regions along a roll direction of the scaffold such that each of the different cell seeding regions forms at least one complete tubular layer after rolling, and the tubular layer located on an outer layer is capable of sequentially wrapping the tubular layer located on the inner layer after rolling, and controlling a thickness ratio of the scaffold in the four different cell seeding regions from thin to thick to be 1:2-80:2-700:2-3,000.

2. The preparation method according to claim 1, wherein the scaffold comprises a dense scaffold and a loose scaffold, and wherein a porosity and a pore size of the loose scaffold are both greater than those of the dense scaffold; preferably, the loose scaffold has a thickness of 20-5,000 μm, a porosity of 90% or more, a pore size of 20-1,000 μm, and a fiber diameter of 0.05-30 μm; the dense scaffold has a thickness of 1-200 μm, a porosity of 59%-90%, a pore size of 0.05-500 μm, and a fiber diameter of 0.05-30 μm; more preferably, a preparation method of the loose scaffold and the dense scaffold comprises any one or more of casting, micro-injection molding, blow molding, extrusion, calendaring, film blowing extrusion, rotational molding, dip coating, 2D printing, hot stamping, melt-blowing, wet spinning, needle spinning, electrospinning, melt spinning, melt-blow spinning, thermal bonding, chemical bonding, spunlacing needle punching, or other non-woven processes; more preferably, materials used for the loose scaffold and the dense scaffold comprise at least one of PGA, PLA, PCL, PLC, PPDO, PHBV, HA, PU, PET, PP, PE, PA, PMMA, PTFE, PES, PEEK, PS, PP, PMMA, PC, PGA-TMC, PGA-CL, chitosan or collagen, or copolymer materials thereof.

3. The preparation method according to claim 1 or 2, wherein the three-dimensional artificial tubular tissue comprises neural tissue, and a method for preparing a scaffold thereof comprises: dividing the rollable scaffold into two different cell seeding regions from the rolling start end to the rolling termination end, and controlling the thickness ratio of the scaffold in the two different cell seeding regions from thin to thick to be 1:20-50; preferably, the scaffold of the neural tissue further comprises a double-layered bundled neural structure, wherein the bundled nerves are wrapped by two layers of outer sheath tissue, and a thickness ratio of the bundled nerves from the rolling start end to the rolling termination end is controlled to be 1:12-24; more preferably, prismatic protrusions distributed along an axial direction are further provided in a region of the neural scaffold at the rolling start end, a distribution density of the prismatic protrusions is 10-15 per millimeter, the length is 5-20 mm, each prismatic protrusion is independent of each other and is separated from each other by a dense layer of 1-8 μm.

4. The preparation method according to claim 1 or 2, wherein the three-dimensional artificial tubular tissue comprises a medium-caliber arterial vascular tissue, and a method for preparing a scaffold thereof comprises: dividing the rollable scaffold into two different cell seeding regions from the rolling start end to the rolling termination end, and controlling the thickness ratio of the scaffold in the two different cell seeding regions from thin to thick to be 1:3-75; alternatively, dividing the rollable scaffold into three different cell seeding regions from the rolling start end to the rolling termination end, and controlling the thickness ratio of the scaffold in the three different cell seeding regions from thin to thick to be 1:3-58:3-60 in sequence;

alternatively, preferably, the three-dimensional artificial tubular tissue comprises a small-caliber arterial vascular tissue, and a preparation method of a scaffold thereof comprises: dividing the rollable scaffold into three different cell seeding regions in sequence from the rolling start end to the rolling termination end, and controlling the thickness ratio of the scaffold in the three different cell seeding regions from thin to thick to be 1:2-25:3-48 in sequence;
alternatively, preferably, the three-dimensional artificial tubular tissue comprises a medium-caliber venous vascular tissue, and a preparation method of a scaffold thereof comprises: dividing the rollable scaffold into three different cell seeding regions in sequence from the rolling start end to the rolling termination end, and controlling the thickness ratio of the scaffold in the three different cell seeding regions from thin to thick to be 1:2-38:3-70 in sequence;
alternatively, preferably, the three-dimensional artificial tubular tissue comprises a small-caliber venous vascular tissue, and a preparation method of a scaffold thereof comprises: dividing the rollable scaffold into three different cell seeding regions in sequence from the rolling start end to the rolling termination end, and controlling the thickness ratio of the scaffold in the three different cell seeding regions from thin to thick to be 1:5-50:7-66 in sequence.

5. The preparation method according to claim 1 or 2, wherein the three-dimensional artificial tubular tissue comprises a female urethra tissue, and a method for preparing a scaffold thereof comprises: dividing the rollable scaffold into three different cell seeding regions in sequence from the rolling start end to the rolling termination end, and controlling the thickness ratio of the scaffold in the three different cell seeding regions from thin to thick to be 1:5-27:8-60 in sequence; alternatively, preferably, the three-dimensional artificial tubular tissue comprises a male urethra tissue, and a preparation method of a scaffold thereof comprises: dividing the rollable scaffold into three different cell seeding regions in sequence from the rolling start end to the rolling termination end, and controlling the thickness ratio of the scaffold in the three different cell seeding regions from thin to thick to be 1:4-28:10-66 in sequence.

6. The preparation method according to claim 1 or 2, wherein the three-dimensional artificial tubular tissue comprises a vas deferens tissue, and a method for preparing a scaffold thereof comprises: dividing the rollable scaffold into three different cell seeding regions in sequence from the rolling start end to the rolling termination end, and controlling the thickness ratio of the scaffold in the three different cell seeding regions from thin to thick to be 1:4-700:16-1,000 in sequence;

alternatively, preferably, the three-dimensional artificial tubular tissue comprises a fallopian tube tissue, and a preparation method of a scaffold thereof comprises: dividing the rollable scaffold into four different cell seeding regions in sequence from the rolling start end to the rolling termination end, and controlling the thickness ratio of the scaffold in the four different cell seeding regions from thin to thick to be 1:2-150:3-300:20-1,000 in sequence; more preferably, a villus structure is further provided in the cell seeding regions of the fallopian tube tissue scaffold near the rolling start end, wherein the villus structure is a protrusion with an axial height of 10-11 $\mu$m and a diameter of 0.2-0.3 $\mu$m.

7. The preparation method according to claim 1 or 2, wherein the three-dimensional artificial tubular tissue comprises an intestinal tissue, and a method for preparing a scaffold thereof comprises: dividing the rollable scaffold into four different cell seeding regions in sequence from the rolling start end to the rolling termination end, and controlling the thickness ratio of the scaffold in the four different cell seeding regions from thin to thick to be 1:2-4:2-30:3-35;

preferably, the intestinal tissue comprises small intestine, and protrusions with an axial height of 4-7 mm and a width of 2-3 mm are further provided at a rolling start end region of the small intestine scaffold; more preferably, the protrusions comprise villi and crypts, wherein the villi have a radial length of 1,200-1,600 $\mu$m and an axial width of 300-600 $\mu$m; the crypts have a neck diameter of 2-10 $\mu$m, and a lumen diameter of 40-50 $\mu$m;
preferably, the intestinal tissue comprises large intestine, and crypts are further provided at a rolling start end region of the large intestine scaffold; the crypts have a distribution density of 100 per square millimeter in the inner layer, a radial depth of 1,500-2,200 $\mu$m, and a diameter of 400-500 $\mu$m.

8. The preparation method according to claim 1 or 2, wherein the three-dimensional artificial tubular tissue comprises an esophageal tissue, and a method for preparing a scaffold thereof comprises: dividing the rollable scaffold into four different cell seeding regions from the rolling start end to the rolling termination end, and controlling the thickness ratio of the scaffold in the four different cell seeding regions from thin to thick to be 1:2-100:5-600:20-1,000 in sequence; preferably, the three-dimensional artificial tubular tissue comprises a tracheal tissue, and a method for preparing a scaffold thereof includes: dividing the rollable scaffold into four different cell seeding regions from the rolling start end to the rolling termination end, and controlling the thickness ratio of the scaffold in the four different cell seeding regions from thin to thick to be 1:4-9:15-38:25-56 in sequence; more preferably, a third cell-seeding region of the tracheal tissue scaffold from the rolling start end further comprises a portion made of a polymer material with a mechanical strength higher than 5-10 MPa or of chondrocytes; and more preferably, the portion with high mechanical strength is annular and has at least one opening.

9. A three-dimensional artificial tubular tissue prepared by the preparation method according to any one of claims 1 to 8, preferably, the three-dimensional artificial tubular tissue comprises artificial nerves, artificial arterial blood vessels, artificial venous blood vessels, artificial small intestines, artificial large intestines, artificial esophagus, artificial tracheas, artificial urethras, artificial fallopian tubes and artificial vas deferens; more preferably, the artificial arterial blood vessels comprise medium-caliber arterial blood vessels and small-caliber arterial blood vessels; more preferably, the artificial venous blood vessels comprises medium-caliber venous blood vessels and small-caliber venous blood vessels; and more preferably, the artificial urethras comprise male urethras and female urethras.

10. Use of the three-dimensional artificial tubular tissue prepared by the method according to any one of claims 1 to 8 or the three-dimensional artificial tubular tissue according to claim 9, wherein the three-dimensional artificial tubular tissue is used as a substitute for a damaged tissue or as an *in vivo* implant.

Endothelial cell    Smooth muscle cell    Fibroblast

PGA scaffold

Start end    Termination end

PLA scaffold

Smooth muscle cell
Loose layer
Dense layer

Fibroblast
Endothelial cell

Figure 1

Figure 2

Figure 3

Figure 4

Figure 5

Comparison of Young's Modulus of Blood Vessels

Figure 6

Figure 7

Figure 8

Figure 9

Figure 10

Figure 11

Figure 12

Figure 13

Figure 14

Figure 15

Figure 16

Figure 17

Figure 18

Comparison of Cell Density between Tissues with
Loose- Dense Structure and without Loose-Dense Structure

Figure 19

Figure 20

Intestinal Crypts    Intestinal Villi

Figure 21

Prismatic Protrusions

Figure 22

Figure 23

Figure 24

Figure 25

Figure 26

Figure 27

Figure 28

Figure 29

Figure 30

## INTERNATIONAL SEARCH REPORT

| | International application No. |
|---|---|
| | **PCT/CN2023/104699** |

**A. CLASSIFICATION OF SUBJECT MATTER**

C12N5/071(2010.01)i; A61L27/50(2006.01)i; A61L27/56(2006.01)i; A61L27/40(2006.01)i; A61L27/38(2006.01)i

According to International Patent Classification (IPC) or to both national classification and IPC

**B. FIELDS SEARCHED**

Minimum documentation searched (classification system followed by classification symbols)

C12N A61L

Documentation searched other than minimum documentation to the extent that such documents are included in the fields searched

Electronic data base consulted during the international search (name of data base and, where practicable, search terms used)

CNTXT, ENTXTC, OETXT, DWPI, SIPOABS, CNABS, CNKI, PUBMED, web of knowledge: 人工, 卷, 管, 厚, 比例, 支架, 种植, 接种, 人工血管, 空腔, 成纤维细胞, 内皮细胞, 平滑肌细胞, 组织工程, rolling scaffold, vessel-like structure, vascular, graft, thickness, ratio, length, endothelial cell, smooth muscle cell, fibroblast , cavity, cell seeding, tissue engineering

**C. DOCUMENTS CONSIDERED TO BE RELEVANT**

| Category* | Citation of document, with indication, where appropriate, of the relevant passages | Relevant to claim No. |
|---|---|---|
| A | CN 108653815 A (NATIONAL CENTER FOR NANOSCIENCE AND TECHNOLOGY) 16 October 2018 (2018-10-16) abstract, claims 1-10, and description, paragraphs 45-48, and figures 1-3 | 1-10 |
| A | JP 2019503819 A (BIOSTAGE INC. et al.) 14 February 2019 (2019-02-14) claims 1-25, and description, paragraphs 45, 56 and 67 | 1-10 |
| A | US 2013345794 A1 (KHATIWALA CHIRAG et al.) 26 December 2013 (2013-12-26) abstract, and claims 1-40 | 1-10 |
| A | US 5116309 A (COLL M.E.) 26 May 1992 (1992-05-26) claims 1-9 | 1-10 |
| A | WANG, N.X. et al. "In Vitro Evaluation of Essential Mechanical Properties and Cell Behaviors of a Novel Polylactic-co-Glycolic Acid (PLGA)-Based Tubular Scaffold for Small-Diameter Vascular Tissue Engineering" *Polymers*, Vol. 9, No. (318), 30 July 2017 (2017-07-30), abstract, page 13, paragraph 2, and Supplementary Materials, figures S3, S6 and S14 | 1-10 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **10 November 2023** | **22 November 2023** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/104699** |

**C.      DOCUMENTS CONSIDERED TO BE RELEVANT**

| Category* | Citation of document, with indication, where appropriate, of the relevant passages | Relevant to claim No. |
| --- | --- | --- |
| A | CHENG. S.Y. et al. "Self-Adjusting, Polymeric Multilayered Roll that can Keep the Shapes of the Blood Vessel Scaffolds during Biodegradation" *Advanced Materials*, 17 May 2017 (2017-05-17), abstract, pages 1-6, and figure 1 | 1-10 |
| A | VALDERRAMA-TREVIŇO, A.I. et al. "Tubular Electrospun Scaffolds Tested In Vivo for Tissue Engineering" *International Journal of Research in Medical Sciences,* Vol. 7, No. (2), 28 February 2019 (2019-02-28), pp. 635-643 | 1-10 |

Form PCT/ISA/210 (second sheet) (July 2022)

## INTERNATIONAL SEARCH REPORT

International application No.

**PCT/CN2023/104699**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **10**
because they relate to subject matter not required to be searched by this Authority, namely:

Claim 10 relates to a disease treatment method, which belongs to the cases set out in PCT Rule 39.1(iv) for which no international search is required. In the present report, a search is carried out on the basis of "the use in the preparation of a substitute for damaged tissue or a material of an in-vivo implant".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/CN2023/104699**

| Patent document cited in search report | | | Publication date (day/month/year) | Patent family member(s) | | | Publication date (day/month/year) |
|---|---|---|---|---|---|---|---|
| CN | 108653815 | A | 16 October 2018 | None | | | |
| JP | 2019503819 | A | 14 February 2019 | None | | | |
| US | 2013345794 | A1 | 26 December 2013 | CA | 2793205 | A1 | 22 September 2011 |
| | | | | CA | 2793205 | C | 05 January 2016 |
| | | | | US | 2020188082 | A1 | 18 June 2020 |
| | | | | HK | 1159682 | A1 | 03 August 2012 |
| | | | | KR | 20130007610 | A | 18 January 2013 |
| | | | | JP | 2016052527 | A | 14 April 2016 |
| | | | | EP | 2547288 | A2 | 23 January 2013 |
| | | | | EP | 2547288 | A4 | 26 February 2014 |
| | | | | EP | 2547288 | B1 | 03 July 2019 |
| | | | | EP | 2547288 | B8 | 14 August 2019 |
| | | | | WO | 2011116125 | A2 | 22 September 2011 |
| | | | | WO | 2011116125 | A3 | 19 January 2012 |
| | | | | KR | 20150038665 | A | 08 April 2015 |
| | | | | KR | 101735971 | B1 | 29 May 2017 |
| | | | | IL | 221897 | A | 30 March 2017 |
| | | | | RU | 2012142992 | A | 27 April 2014 |
| | | | | RU | 2522966 | C2 | 20 July 2014 |
| | | | | GB | 201203622 | D0 | 18 April 2012 |
| | | | | GB | 2489081 | A | 19 September 2012 |
| | | | | GB | 2489081 | B | 27 March 2013 |
| | | | | JP | 2013538064 | A | 10 October 2013 |
| | | | | JP | 5950899 | B2 | 13 July 2016 |
| | | | | US | 2023320837 | A1 | 12 October 2023 |
| | | | | AU | 2011227282 | A1 | 08 November 2012 |
| | | | | AU | 2011227282 | B2 | 19 September 2013 |
| | | | | GB | 201008781 | D0 | 14 July 2010 |
| | | | | GB | 2478801 | A | 21 September 2011 |
| | | | | GB | 2478801 | B | 30 May 2012 |
| US | 5116309 | A | 26 May 1992 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102884172 B
- CN 106085847 B
- CN 108653815 B

**Non-patent literature cited in the description**

- Clinical analysis of young adult patients with ruptured intracranial aneurysms: a single-center study of 113 consecutive patients. *J Cerebrovasc Endovasc Neurosurg.*, 21 September 2020, vol. 22 (3), 127-133
- **STANKUS J J** ; **SOLETTI L** ; **FUJIMOTO K et al.** Fabrication of cell microintegrated blood vessel constructs through electrohydrodynamic atomization[J].. *Biomaterials*, 2007, vol. 28 (17), 2738-2746
- **ZHENG W** ; **WANG Z** ; **SONG L et al.** Endothelialization and patency of RGD-functionalized vascular grafts in a rabbit carotid artery model[J].. *Biomaterials*, 2012, vol. 33 (10), 2880-2891
- **TEEBKEN O E** ; **HAVERICH A**. Tissue engineering of small diameter vascular grafts[J].. *European Journal of Vascular and Endovascular Surgery*, 2002, vol. 23 (6), 475-485
- **KIM M J** ; **KIM JH** ; **YI G et al.** In vitro and in vivo application of PLGA nanofiber for artificial blood vessel[J].. *Macromolecular Research*, 2008, vol. 16, 345-352
- **WANG W** ; **HU J** ; **HE C et al.** Heparinized PLLA/PLCL nanofibrous scaffold for potential engineering of small-diameter blood vessel: Tunable elasticity and anticoagulation property[J].. *Journal of Biomedical Materials Research Part A*, 2015, vol. 103 (5), 1784-1797
- **L'HEUREUX N** ; **DUSSERRE N** ; **KONIG G et al.** Human tissue-engineered blood vessels for adult arterial revascularization[J].. *Nature Medicine*, 2006, vol. 12 (3), 361-365
- **TALACUA H** ; **SMITS AIPM** ; **MUYLAERT DEP et al.** In situ tissue engineering of functionalsmall-diameter blood vessels by host circulating cells only[J].. *Tissue Engineering Part A*, 2015, vol. 21 (19-20), 2583-2594
- Clinical analysis of young adult patients with ruptured intracranial aneurysms: a single-center study of 113 consecutive patients.. *J Cerebrovasc Endovasc Neurosurg.*, 21 September 2020, vol. 22 (3), 127-133
- **NICOLASL'HEUREUX**. *A completely biological tissue-engineered human blood vessel*, 1998